# EUROPÄISCHE PATENTSCHRIFT

(11) **EP 1 641 830 B1**
(45) Veröffentlichungstag und Bekanntmachung des Hinweises auf die Patenterteilung: **09.06.2010**
(21) Anmeldenummer: 04740875.2
(22) Anmeldetag: 09.07.2004
(51) Int. Cl.: C07K 16/44, C12N 15/13, C12N 5/10

(54) **EMITTER-BINDENDE PEPTIDE, DIE EINE VERÄNDERUNG DER SPEKTRALEN EMISSIONSEIGENSCHAFTEN DES EMITTERS BEWIRKEN**
EMITTER-BINDING PEPTIDES CAUSING A CHANGE IN THE SPECTRAL EMISSION PROPERTIES OF THE EMITTER
PEPTIDES QUI SE LIENT A UN EMETTEUR ET QUI PROVOQUENT UNE MODIFICATION DES PROPRIETES SPECTRALES D'EMISSION DE CET EMETTEUR

(30) Priorität: 09.07.2003 DE 10331054; 16.07.2003 US 487234 P
(43) Veröffentlichungstag der Anmeldung: 05.04.2006
(73) Patentinhaber: Bayer Schering Pharma Aktiengesellschaft, 13353 Berlin (DE); MorphoSys AG, 82152 Martinsried/München (DE)
(72) Erfinder: SCHIRNER, Michael, 13158 Berlin (DE); LICHA, Kai, 14612 Falkensee (DE); MENRAD, Andreas, 16515 Oranienburg (DE); URLINGER, Stefanie, 90469 Nürnberg (DE); HANEL, Cornelia, 80469 München (DE); BROCKS, Bodo, 82205 Gilching (DE)
(74) Vertreter: Kilger, Ute
(86) Internationale Anmeldenummer: PCT/EP2004/007596
(87) Internationale Veröffentlichungsnummer: WO 2005/005483

(56) Entgegenhaltungen:
- EP-A- 1 136 556
- WO-A-02/22629
- WO-A-2004/027424
- US-A- 5 569 587
- US-A1- 2003 065 150
- US-B1- 6 492 160
- SPIEGEL S ET AL: "DIRECT VISUALIZATION OF REDISTRIBUTION AND CAPPING OF FLUORESCENT GANGLIOSIDES ON LYMPHOCYTES" JOURNAL OF CELL BIOLOGY, Bd. 99, Nr. 5, 1984, Seiten 1575-1581, XP002304051 ISSN: 0021-9525
- SIMEONOV A ET AL: "BLUE-FLUORESCENT ANTIBODIES" SCIENCE, AMERICAN ASSOCIATION FOR THE ADVANCEMENT OF SCIENCE,, US, Bd. 290, 13. Oktober 2000 (2000-10-13), Seiten 307-313, XP002906656 ISSN: 0036-8075
- WHITLOW M ET AL: "MULTIVALENT FVS: CHARACTERIZATION OF SINGLE-CHAIN FV OLIGOMERS AND PREPARATION OF A BISPECIFIC FV" PROTEIN ENGINEERING, OXFORD UNIVERSITY PRESS, SURREY, GB, Bd. 7, Nr. 8, 1. August 1994 (1994-08-01), Seiten 1017-1026, XP000445076 ISSN: 0269-2139

### SEQUENCE LISTING

<110> Schering AG
   MorphoSys AG
<120> Emitter-bindende Peptide, die eine Veränderung der spektralen Emissionseigenschaften des Emitters bewirken
<130> S30460PCT
<150> DE 103 31 054.1
   <151> 2003-07-09
<150> US 60/487,234
   <151> 2003-07-16
<160> 40
<170> PatentIn version 3.2
<210> 1
   <211> 252
   <212> PRT
   <213> Artificial
<220>
   <223> Fab fragment MOR02628 VH-CH
<400> 1
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp Ser Trp Ile Arg Gln Ser Pro Gly Arg Gly Leu Glu
           35                  40                  45
   Trp Leu Gly Arg Ile Tyr Tyr Arg Ser Lys Trp Tyr Asn Asp Tyr Ala
       50                  55                  60
   Val Ser Val Lys Ser Arg Ile Thr Ile Asn Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Thr Ser Phe Tyr Gln Lys Leu Phe Phe Ile Ala
               100                 105                 110
   Phe Asp His Trp Gly Gln Gly Thr Leu Val Thr Val Ser Ser Ala Ser
           115                 120                 125
   Thr Lys Gly Pro Ser Val Phe Pro Leu Ala Pro Ser Ser Lys Ser Thr
   130 135 140
   Ser Gly Gly Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Ser Leu Gly Thr Gln Thr Tyr Ile
   195 200 205
   Cys Asn Val Asn His Lys Pro Ser Asn Thr Lys Val Asp Lys Lys Val
   210 215 220
   Glu Pro Lys Ser Glu Phe Glu Gln Lys Leu Ile Ser Glu Glu Asp Leu
   225 230 235 240
   Asn Gly Ala Pro Trp Ser His Pro Gln Phe Glu Lys
   245 250
<210> 2
   <211> 216
   <212> PRT
   <213> Artificial
<220>
   <223> Fab fragment MOR02628 VL-CL
<400> 2
   Asp Ile Ala Leu Thr Gln Pro Ala Ser Val Ser Gly Ser Pro Gly Gln
   1 5 10 15
   Ser Ile Thr Ile Ser Cys Thr Gly Thr Ser Ser Asp Gly Gly Ala Tyr
   20 25 30
   His Tyr Val Ser Trp Tyr Gln Gln His Pro Gly Lys Ala Pro Lys Leu
   35 40 45
   Met Ile Tyr Gly Val Ile Tyr Arg Pro Ser Gly Val Ser Asn Arg Phe
   50 55 60
   Ser Gly Ser Lys Ser Gly Asn Thr Ala Ser Leu Thr Ile Ser Gly Leu
   65 70 75 80
   Gln Ala Glu Asp Glu Ala Asp Tyr Tyr Cys Ala Ala Trp Asp Phe Arg
   85 90 95
   Lys Arg Leu Asn Val Phe Gly Gly Gly Thr Lys Leu Thr Val Leu Gly
   100 105 110
   Gln Pro Lys Ala Ala Pro Ser Val Thr Leu Phe Pro Pro Ser Ser Glu
   115 120 125
   Glu Leu Gln Ala Asn Lys Ala Thr Leu Val Cys Leu Ile Ser Asp Phe
   130 135 140
   Tyr Pro Gly Ala Val Thr Val Ala Trp Lys Ala Asp Ser Ser Pro Val
   145 150 155 160
   Lys Ala Gly Val Glu Thr Thr Thr Pro Ser Lys Gln Ser Asn Asn Lys
   165 170 175
   Tyr Ala Ala Ser Ser Tyr Leu Ser Leu Thr Pro Glu Gln Trp Lys Ser
   180 185 190
   His Arg Ser Tyr Ser Cys Gln Val Thr His Glu Gly Ser Thr Val Glu
   195 200 205
   Lys Thr Val Ala Pro Thr Glu Ala
   210 215
<210> 3
   <211> 762
   <212> DNA
   <213> Artificial
<220>
   <223> DNA coding for Fab fragment MOR02628 VH-CH
<400> 3
   caggtgcaat tgcaacagtc tggtccgggc ctggtgaaac cgagccaaac cctgagcctg 60
   acctgtgcga tttccggaga tagcgtgagc tctaattctg ctgcttggtc ttggattcgc 120
   cagtctcctg ggcgtggcct cgagtggctg ggccgtatct attatcgtag caagtggtat 180
   aacgattatg cggtgagcgt gaaaagccgg attaccatca acccggatac ttcgaaaaac 240
   cagtttagcc tgcaactgaa cagcgtgacc ccggaagata cggccgtgta ttattgcgcg 300
   cgtacttctt tttatcagaa gctttttttt attgcttttg atcattgggg ccaaggcacc 360
   ctggtgacgg ttagctcagc gtcgaccaaa ggtccaagcg tgtttccgct ggctccgagc 420
   agcaaaagca ccagcggcgg cacggctgcc ctgggctgcc tggttaaaga ttatttcccg 480
   gaaccagtca ccgtgagctg gaacagcggg gcgctgacca gcggcgtgca tacctttccg 540
   gcggtgctgc aaagcagcgg cctgtatagc ctgagcagcg ttgtgaccgt gccgagcagc 600
   agcttaggca ctcagaccta tatttgcaac gtgaaccata aaccgagcaa caccaaagtg 660
   gataaaaaag tggaaccgaa aagcgaattc gagcagaagc tgatctctga ggaggatctg 720
   aacggcgcgc cgtggagcca cccgcagttt gaaaaatgat aa 762
<210> 4
   <211> 654
   <212> DNA
   <213> Artificial
<220>
   <223> DNA coding for Fab fragment MOR02628 VL-CL
<400> 4
   gatatcgcac tgacccagcc agcttcagtg agcggctcac caggtcagag cattaccatc      60
   tcgtgtacgg gtactagcag cgatggtggt gcttatcatt atgtgtcttg gtaccagcag     120
   catcccggga aggcgccgaa acttatgatt tatggtgtta tttatcgtcc ctcaggcgtg     180
   agcaaccgtt ttagcggatc caaaagcggc aacaccgcga gcctgaccat tagcggcctg     240
   caagcggaag acgaagcgga ttattattgc gctgcttggg attttcgtaa gcgtcttaat     300
   gtgtttggcg gcggcacgaa gttaaccgtt cttggccagc cgaaagccgc accgagtgtg     360
   acgctgtttc cgccgagcag cgaagaattg caggcgaaca aagcgaccct ggtgtgcctg     420
   attagcgact tttatccggg agccgtgaca gtggcctgga aggcagatag cagccccgtc     480
   aaggcgggag tggagaccac cacaccctcc aaacaaagca acaacaagta cgcggccagc     540
   agctatctga gcctgacgcc tgagcagtgg aagtcccaca gaagctacag ctgccaggtc     600
   acgcatgagg ggagcaccgt ggaaaaaacc gttgcgccga ctgaggcctg ataa           654
<210> 5
   <211> 245
   <212> PRT
   <213> Artificial
<220>
   <223> Fab fragment MOR02965 VH-CH
<400> 5
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Asn Ser Tyr
               20                  25                  30
   Ala Ile Ser Trp Val Arg Gln Ala Pro Gly Gln Gly Leu Glu Trp Met
           35                  40                  45
   Gly Gly Ile His Pro Tyr Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Arg Trp Arg Tyr Arg Trp Gly Phe Asp Ile Trp Gly Gln Gly
   100 105 110
   Thr Leu Val Thr Val Ser Ser Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Lys Val Glu Pro Lys Ser Glu Phe Glu
   210 215 220
   Gln Lys Leu Ile Ser Glu Glu Asp Leu Asn Gly Ala Pro Trp Ser His
   225 230 235 240
   Pro Gln Phe Glu Lys
   245
<210> 6
   <211> 214
   <212> PRT
   <213> Artificial
<220>
   <223> Fab fragment MOR02965 VL-CL
<400> 6
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Tyr
               20                  25                  30
   Tyr Val Tyr Trp Tyr Gln Gln Leu Pro Gly Thr Ala Pro Lys Leu Leu
           35                  40                  45
   Ile Tyr Gly Asn Ser Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ser Ser Trp Asp Ser Ser Phe
                   85                  90                  95
   Ser Trp Val Phe Gly Gly Gly Thr Lys Leu Thr Val Leu Gly Gln Pro
               100                 105                 110
   Lys Ala Ala Pro Ser Val Thr Leu Phe Pro Pro Ser Ser Glu Glu Leu
           115                 120                 125
   Gln Ala Asn Lys Ala Thr Leu Val Cys Leu Ile Ser Asp Phe Tyr Pro
       130                 135                 140
   Gly Ala Val Thr Val Ala Trp Lys Ala Asp Ser Ser Pro Val Lys Ala
   145                 150                 155                 160
   Gly Val Glu Thr Thr Thr Pro Ser Lys Gln Ser Asn Asn Lys Tyr Ala
                   165                 170                 175
   Ala Ser Ser Tyr Leu Ser Leu Thr Pro Glu Gln Trp Lys Ser His Arg
               180                 185                 190
   Ser Tyr Ser Cys Gln Val Thr His Glu Gly Ser Thr Val Glu Lys Thr
           195                 200                 205
   Val Ala Pro Thr Glu Ala
       210
<210> 7
   <211> 741
   <212> DNA
   <213> Artificial
<220>
   <223> DNA coding for Fab fragment MOR02965 VH-CH
<400> 7
   caggtgcaat tggttcagtc tggcgcggaa gtgaaaaaac cgggcagcag cgtgaaagtg      60
   agctgcaaag cctccggagg cacttttaat tcttatgcta tttcttgggt gcgccaagcc     120
   cctgggcagg gtctcgagtg gatgggcggt atccatccgt attttggcac tgcgaattac     180
   gcgcagaagt ttcagggccg ggtgaccatt accgcggatg aaagcaccag caccgcgtat     240
   atggaactga gcagcctgcg tagcgaagat acggccgtgt attattgcgc gcgtcgttgg     300
   cgttatcgtt ggggttttga tatttggggc caaggcaccc tggtgacggt tagctcagcg     360
   tcgaccaaag gtccaagcgt gtttccgctg gctccgagca gcaaaagcac cagcggcggc     420
   acggctgccc tgggctgcct ggttaaagat tatttcccgg aaccagtcac cgtgagctgg     480
   aacagcgggg cgctgaccag cggcgtgcat acctttccgg cggtgctgca aagcagcggc     540
   ctgtatagcc tgagcagcgt tgtgaccgtg ccgagcagca gcttaggcac tcagacctat     600
   atttgcaacg tgaaccataa accgagcaac accaaagtgg ataaaaaagt ggaaccgaaa     660
   agcgaattcg agcagaagct gatctctgag gaggatctga acggcgcgcc gtggagccac     720
   ccgcagtttg aaaaatgata a                                               741
<210> 8
   <211> 648
   <212> DNA
   <213> Artificial
<220>
   <223> DNA coding for Fab fragment MOR02965 VL-CL
<400> 8
   gatatcgtgc tgacccagcc gccttcagtg agtggcgcac caggtcagcg tgtgaccatc     60
   tcgtgtagcg gcagcagcag caacattggt tcttattatg tgtattggta ccagcagttg    120
   cccgggacgg cgccgaaact tctgatttat ggtaattctc agcgtccctc aggcgtgccg    180
   gatcgtttta gcggatccaa aagcggcacc agcgcgagcc ttgcgattac gggcctgcaa    240
   agcgaagacg aagcggatta ttattgctct tcttgggatt cttctttttc ttgggtgttt    300
   ggcggcggca cgaagttaac cgttcttggc cagccgaaag ccgcaccgag tgtgacgctg    360
   tttccgccga gcagcgaaga attgcaggcg aacaaagcga ccctggtgtg cctgattagc    420
   gacttttatc cgggagccgt gacagtggcc tggaaggcag atagcagccc cgtcaaggcg    480
   ggagtggaga ccaccacacc ctccaaacaa agcaacaaca agtacgcggc cagcagctat    540
   ctgagcctga cgcctgagca gtggaagtcc cacagaagct acagctgcca ggtcacgcat    600
   gaggggagca ccgtggaaaa aaccgttgcg ccgactgagg cctgataa                 648
<210> 9
   <211> 246
   <212> PRT
   <213> Artificial
<220>
   <223> Fab fragment MOR02977 VH-CH
<400> 9
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Asn Tyr
               20                  25                  30
   Ala Ile Asn Trp Val Arg Gln Ala Pro Gly Gln Gly Leu Glu Trp Met
           35                  40                  45
   Gly Asn Ile Glu Pro Tyr Phe Gly Thr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Tyr Phe Met Ser Tyr Lys His Leu Ser Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Glu Phe
       210                 215                 220
   Glu Gln Lys Leu Ile Ser Glu Glu Asp Leu Asn Gly Ala Pro Trp Ser
   225                 230                 235                 240
   His Pro Gln Phe Glu Lys
   245
<210> 10
   <211> 216
   <212> PRT
   <213> Artificial
<220>
   <223> Fab fragment MOR02977 VL-CL
<400> 10
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Thr Thr Arg
                   85                  90                  95
   Pro Leu Asn Arg Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 11
   <211> 744
   <212> DNA
   <213> Artificial
<220>
   <223> DNA coding for Fab fragment MOR02977 VH-CH
<400> 11
   caggtgcaat tggttcagtc tggcgcggaa gtgaaaaaac cgggcagcag cgtgaaagtg      60
   agctgcaaag cctccggagg cactttttct aattatgcta ttaattgggt gcgccaagcc    120
   cctgggcagg gtctcgagtg gatgggcaat atcgagccgt attttggcac tgcgaattac    180
   gcgcagaagt ttcagggccg ggtgaccatt accgcggatg aaagcaccag caccgcgtat    240
   atggaactga gcagcctgcg tagcgaagat acggccgtgt attattgcgc gcgttatttt    300
   atgtcttata agcatctttc tgattattgg ggccaaggca ccctggtgac ggttagctca    360
   gcgtcgacca aaggtccaag cgtgtttccg ctggctccga gcagcaaaag caccagcggc    420
   ggcacggctg ccctgggctg cctggttaaa gattatttcc cggaaccagt caccgtgagc    480
   tggaacagcg gggcgctgac cagcggcgtg catacctttc cggcggtgct gcaaagcagc    540
   ggcctgtata gcctgagcag cgttgtgacc gtgccgagca gcagcttagg cactcagacc    600
   tatatttgca acgtgaacca taaaccgagc aacaccaaag tggataaaaa agtggaaccg    660
   aaaagcgaat tcgagcagaa gctgatctct gaggaggatc tgaacggcgc gccgtggagc    720
   cacccgcagt ttgaaaaatg ataa                                            744
<210> 12
   <211> 654
   <212> DNA
   <213> Artificial
<220>
   <223> DNA coding for Fab fragment MOR02977 VL-CL
<400> 12
   gatatcgcac tgacccagcc agcttcagtg agcggctcac caggtcagag cattaccatc      60
   tcgtgtacgg gtactagcag cgatgttggt tctaataatt atgtgtcttg gtaccagcag    120
   catcccggga aggcgccgaa acttatgatt tatggtggtt ctaatcgtcc ctcaggcgtg    180
   agcaaccgtt ttagcggatc caaaagcggc aacaccgcga gcctgaccat tagcggcctg    240
   caagcggaag acgaagcgga ttattattgc cagtcttgga ctactcgtcc tcttaatcgt    300
   gtgtttggcg gcggcacgaa gttaaccgtt cttggccagc cgaaagccgc accgagtgtg     360
   acgctgtttc cgccgagcag cgaagaattg caggcgaaca aagcgaccct ggtgtgcctg     420
   attagcgact tttatccggg agccgtgaca gtggcctgga aggcagatag cagccccgtc     480
   aaggcgggag tggagaccac cacaccctcc aaacaaagca acaacaagta cgcggccagc     540
   agctatctga gcctgacgcc tgagcagtgg aagtcccaca gaagctacag ctgccaggtc     600
   acgcatgagg ggagcaccgt ggaaaaaacc gttgcgccga ctgaggcctg ataa           654
<210> 13
   <211> 241
   <212> PRT
   <213> Artificial
<220>
   <223> Fab fragment MOR02969 VH-CH
<400> 13
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp His
               20                  25                  30
   Trp Met Ser Trp Val Arg Gln Ala Pro Gly Lys Gly Leu Glu Trp Val
           35                  40                  45
   Ser Asn Ile Ser Gly Ser Ser Ser Asn Thr Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Gly Met Ala Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Glu Phe Glu Gln Lys Leu Ile
       210                 215                 220
   Ser Glu Glu Asp Leu Asn Gly Ala Pro Trp Ser His Pro Gln Phe Glu
   225                 230                 235                 240
   Lys
<210> 14
   <211> 213
   <212> PRT
   <213> Artificial
<220>
   <223> Fab fragment MOR02969 VL-CL
<400> 14
   Asp Ile Glu Leu Thr Gln Pro Pro Ser Val Ser Val Ala Pro Gly Gln
   1 5 10 15
   Thr Ala Arg Ile Ser Cys Ser Gly Asp Ser Ile Arg Ser Lys Tyr Val
   20 25 30
   His Trp Tyr Gln Gln Lys Pro Gly Gln Ala Pro Val Leu Val Ile Tyr
   35 40 45
   Arg Asp Asn Asn Arg Pro Ser Gly Ile Pro Glu Arg Phe Ser Gly Ser
   50 55 60
   Asn Ser Gly Asn Thr Ala Thr Leu Thr Ile Ser Gly Thr Gln Ala Glu
   65 70 75 80
   Asp Glu Ala Asp Tyr Tyr Cys Ser Ser Tyr Thr Tyr Arg Val Gly Gly
   85 90 95
   Met Val Phe Gly Gly Gly Thr Lys Leu Thr Val Leu Gly Gln Pro Lys
   100 105 110
   Ala Ala Pro Ser Val Thr Leu Phe Pro Pro Ser Ser Glu Glu Leu Gln
   115 120 125
   Ala Asn Lys Ala Thr Leu Val Cys Leu Ile Ser Asp Phe Tyr Pro Gly
   130 135 140
   Ala Val Thr Val Ala Trp Lys Ala Asp Ser Ser Pro Val Lys Ala Gly
   145 150 155 160
   Val Glu Thr Thr Thr Pro Ser Lys Gln Ser Asn Asn Lys Tyr Ala Ala
   165 170 175
   Ser Ser Tyr Leu Ser Leu Thr Pro Glu Gln Trp Lys Ser His Arg Ser
   180 185 190
   Tyr Ser Cys Gln Val Thr His Glu Gly Ser Thr Val Glu Lys Thr Val
   195 200 205
   Ala Pro Thr Glu Ala
   210
<210> 15
   <211> 729
   <212> DNA
   <213> Artificial
<220>
   <223> DNA coding for Fab fragment MOR02969 VH-CH
<400> 15
   caggtgcaat tggtggaaag cggcggcggc ctggtgcaac cgggcggcag cctgcgtctg      60
   agctgcgcgg cctccggatt taccttttct gatcattgga tgtcttgggt gcgccaagcc    120
   cctgggaagg gtctcgagtg ggtgagcaat atctctggtt cttctagcaa taccaattat     180
   gcggatagcg tgaaaggccg ttttaccatt tcacgtgata attcgaaaaa caccctgtat     240
   ctgcaaatga acagcctgcg tgcggaagat acggccgtgt attattgcgc gcgtggttat     300
   ggtatggctt attggggcca aggcaccctg gtgacggtta gctcagcgtc gaccaaaggt     360
   ccaagcgtgt ttccgctggc tccgagcagc aaaagcacca gcggcggcac ggctgccctg     420
   ggctgcctgg ttaaagatta tttcccggaa ccagtcaccg tgagctggaa cagcggggcg     480
   ctgaccagcg gcgtgcatac ctttccggcg gtgctgcaaa gcagcggcct gtatagcctg     540
   agcagcgttg tgaccgtgcc gagcagcagc ttaggcactc agacctatat ttgcaacgtg     600
   aaccataaac cgagcaacac caaagtggat aaaaaagtgg aaccgaaaag cgaattcgag     660
   cagaagctga tctctgagga ggatctgaac ggcgcgccgt ggagccaccc gcagtttgaa     720
   aaatgataa                                                             729
<210> 16
   <211> 645
   <212> DNA
   <213> Artificial
<220>
   <223> DNA coding for Fab fragment MOR02969 VL-CL
<400> 16
   gatatcgaac tgacccagcc gccttcagtg agcgttgcac caggtcagac cgcgcgtatc    60
   tcgtgtagcg gcgattctat tcgttctaag tatgttcatt ggtaccagca gaaacccggg   120
   caggcgccag ttcttgtgat ttatcgtgat aataatcgtc cctcaggcat cccggaacgc   180
   tttagcggat ccaacagcgg caacaccgcg accctgacca ttagcggcac tcaggcggaa   240
   gacgaagcgg attattattg ctcttcttat acttataggg ttggtggtat ggtgtttggc   300
   ggcggcacga agttaaccgt tcttggccag ccgaaagccg caccgagtgt gacgctgttt   360
   ccgccgagca gcgaagaatt gcaggcgaac aaagcgaccc tggtgtgcct gattagcgac   420
   ttttatccgg gagccgtgac agtggcctgg aaggcagata gcagccccgt caaggcggga   480
   gtggagacca ccacaccctc caaacaaagc aacaacaagt acgcggccag cagctatctg   540
   agcctgacgc ctgagcagtg gaagtcccac agaagctaca gctgccaggt cacgcatgag   600
   gggagcaccg tggaaaaaac cgttgcgccg actgaggcct gataa                   645
<210> 17
   <211> 214
   <212> PRT
   <213> Artificial
<220>
   <223> Fab fragment MOR03263 VL-CL
<400> 17
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Tyr
               20                  25                  30
   Tyr Val Tyr Trp Tyr Gln Gln Leu Pro Gly Thr Ala Pro Lys Leu Leu
           35                  40                  45
   Ile Tyr Gly Asn Ser Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ser Ser Trp Asp Val Ser Leu
                   85                  90                  95
   Glu Trp Val Phe Gly Gly Gly Thr Lys Leu Thr Val Leu Gly Gln Pro
               100                 105                 110
   Lys Ala Ala Pro Ser Val Thr Leu Phe Pro Pro Ser Ser Glu Glu Leu
           115                 120                 125
   Gln Ala Asn Lys Ala Thr Leu Val Cys Leu Ile Ser Asp Phe Tyr Pro
       130                 135                 140
   Gly Ala Val Thr Val Ala Trp Lys Ala Asp Ser Ser Pro Val Lys Ala
   145                 150                 155                 160
   Gly Val Glu Thr Thr Thr Pro Ser Lys Gln Ser Asn Asn Lys Tyr Ala
                   165                 170                 175
   Ala Ser Ser Tyr Leu Ser Leu Thr Pro Glu Gln Trp Lys Ser His Arg
               180                 185                 190
   Ser Tyr Ser Cys Gln Val Thr His Glu Gly Ser Thr Val Glu Lys Thr
           195                 200                 205
   Val Ala Pro Thr Glu Ala
       210
<210> 18
   <211> 648
   <212> DNA
   <213> Artificial
<220>
   <223> DNA coding for Fab fragment MOR03263 VL-CL
<400> 18
   gatatcgtgc tgacccagcc gccttcagtg agtggcgcac caggtcagcg tgtgaccatc      60
   tcgtgtagcg gcagcagcag caacattggt tcttattatg tgtattggta ccagcagttg     120
   cccgggacgg cgccgaaact tctgatttat ggtaattctc agcgtccctc aggcgtgccg     180
   gatcgtttta gcggatccaa aagcggcacc agcgcgagcc ttgcgattac gggcctgcaa     240
   agcgaagacg aagcggatta ttattgctct tcttgggatg tttctcttga gtgggtgttt     300
   ggcggcggca cgaagttaac cgttcttggc cagccgaaag ccgcaccgag tgtgacgctg     360
   tttccgccga gcagcgaaga attgcaggcg aacaaagcga ccctggtgtg cctgattagc     420
   gacttttatc cgggagccgt gacagtggcc tggaaggcag atagcagccc cgtcaaggcg     480
   ggagtggaga ccaccacacc ctccaaacaa agcaacaaca agtacgcggc cagcagctat     540
   ctgagcctga cgcctgagca gtggaagtcc cacagaagct acagctgcca ggtcacgcat     600
   gaggggagca ccgtggaaaa aaccgttgcg ccgactgagg cctgataa                  648
<210> 19
   <211> 214
   <212> PRT
   <213> Artificial
<220>
   <223> Fab fragment MOR03325 VL-CL
<400> 19
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Tyr
               20                  25                  30
   Tyr Val Tyr Trp Tyr Gln Gln Leu Pro Gly Thr Ala Pro Lys Leu Leu
           35                  40                  45
   Ile Tyr Gly Asn Ser Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Ala Ser Trp Asp Lys Ser Leu
                   85                  90                  95
   Gln Trp Val Phe Gly Gly Gly Thr Lys Leu Thr Val Leu Gly Gln Pro
               100                 105                 110
   Lys Ala Ala Pro Ser Val Thr Leu Phe Pro Pro Ser Ser Glu Glu Leu
           115                 120                 125
   Gln Ala Asn Lys Ala Thr Leu Val Cys Leu Ile Ser Asp Phe Tyr Pro
       130                 135                 140
   Gly Ala Val Thr Val Ala Trp Lys Ala Asp Ser Ser Pro Val Lys Ala
   145                 150                 155                 160
   Gly Val Glu Thr Thr Thr Pro Ser Lys Gln Ser Asn Asn Lys Tyr Ala
                   165                 170                 175
   Ala Ser Ser Tyr Leu Ser Leu Thr Pro Glu Gln Trp Lys Ser His Arg
               180                 185                 190
   Ser Tyr Ser Cys Gln Val Thr His Glu Gly Ser Thr Val Glu Lys Thr
           195                 200                 205
   Val Ala Pro Thr Glu Ala
       210
<210> 20
   <211> 648
   <212> DNA
   <213> Artificial
<220>
   <223> DNA coding for Fab fragment MOR03325 VL-CL
<400> 20
   gatatcgtgc tgacccagcc gccttcagtg agtggcgcac caggtcagcg tgtgaccatc      60
   tcgtgtagcg gcagcagcag caacattggt tcttattatg tgtattggta ccagcagttg     120
   cccgggacgg cgccgaaact tctgatttat ggtaattctc agcgtccctc aggcgtgccg     180
   gatcgtttta gcggatccaa aagcggcacc agcgcgagcc ttgcgattac gggcctgcaa     240
   agcgaagacg aagcggatta ttattgcgct tcttgggata agtctcttca gtgggtgttt     300
   ggcggcggca cgaagttaac cgttcttggc cagccgaaag ccgcaccgag tgtgacgctg     360
   tttccgccga gcagcgaaga attgcaggcg aacaaagcga ccctggtgtg cctgattagc     420
   gacttttatc cgggagccgt gacagtggcc tggaaggcag atagcagccc cgtcaaggcg     480
   ggagtggaga ccaccacacc ctccaaacaa agcaacaaca agtacgcggc cagcagctat     540
   ctgagcctga cgcctgagca gtggaagtcc cacagaagct acagctgcca ggtcacgcat     600
   gaggggagca ccgtggaaaa aaccgttgcg ccgactgagg cctgataa                  648
<210> 21
   <211> 216
   <212> PRT
   <213> Artificial
<220>
   <223> Fab fragment MOR03201 VL-CL
<400> 21
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
   50 55 60
   Ser Gly Ser Lys Ser Gly Asn Thr Ala Ser Leu Thr Ile Ser Gly Leu
   65 70 75 80
   Gln Ala Glu Asp Glu Ala Asp Tyr Tyr Cys Ser Ser Trp Thr Ser Tyr
   85 90 95
   Phe His Ile Arg Val Phe Gly Gly Gly Thr Lys Leu Thr Val Leu Gly
   100 105 110
   Gln Pro Lys Ala Ala Pro Ser Val Thr Leu Phe Pro Pro Ser Ser Glu
   115 120 125
   Glu Leu Gln Ala Asn Lys Ala Thr Leu Val Cys Leu Ile Ser Asp Phe
   130 135 140
   Tyr Pro Gly Ala Val Thr Val Ala Trp Lys Ala Asp Ser Ser Pro Val
   145 150 155 160
   Lys Ala Gly Val Glu Thr Thr Thr Pro Ser Lys Gln Ser Asn Asn Lys
   165 170 175
   Tyr Ala Ala Ser Ser Tyr Leu Ser Leu Thr Pro Glu Gln Trp Lys Ser
   180 185 190
   His Arg Ser Tyr Ser Cys Gln Val Thr His Glu Gly Ser Thr Val Glu
   195 200 205
   Lys Thr Val Ala Pro Thr Glu Ala
   210 215
<210> 22
   <211> 654
   <212> DNA
   <213> Artificial
<220>
   <223> DNA coding for Fab fragment MOR03201 VL-CL
<400> 22
   gatatcgcac tgacccagcc agcttcagtg agcggctcac caggtcagag cattaccatc      60
   tcgtgtacgg gtactagcag cgatgttggt tctaataatt atgtgtcttg gtaccagcag     120
   catcccggga aggcgccgaa acttatgatt tatggtggtt ctaatcgtcc ctcaggcgtg     180
   agcaaccgtt ttagcggatc caaaagcggc aacaccgcga gcctgaccat tagcggcctg     240
   caagcggaag acgaagcgga ttattattgc tcttcttgga cttcttattt tcatattcgt     300
   gtgtttggcg gcggcacgaa gttaaccgtt cttggccagc cgaaagccgc accgagtgtg     360
   acgctgtttc cgccgagcag cgaagaattg caggcgaaca aagcgaccct ggtgtgcctg     420
   attagcgact tttatccggg agccgtgaca gtggcctgga aggcagatag cagccccgtc     480
   aaggcgggag tggagaccac cacaccctcc aaacaaagca acaacaagta cgcggccagc     540
   agctatctga gcctgacgcc tgagcagtgg aagtcccaca gaagctacag ctgccaggtc     600
   acgcatgagg ggagcaccgt ggaaaaaacc gttgcgccga ctgaggcctg ataa           654
<210> 23
   <211> 216
   <212> PRT
   <213> Artificial
<220>
   <223> Fab fragment MOR03267 VL-CL
<400> 23
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ala Trp Asp Ser Asn
                   85                  90                  95
   Phe Lys Asn Arg Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                      165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 24
   <211> 654
   <212> DNA
   <213> Artificial
<220>
   <223> DNA coding for Fab fragment MOR03267 VL-CL
<400> 24
   gatatcgcac tgacccagcc agcttcagtg agcggctcac caggtcagag cattaccatc      60
   tcgtgtacgg gtactagcag cgatgttggt tctaataatt atgtgtcttg gtaccagcag     120
   catcccggga aggcgccgaa acttatgatt tatggtggtt ctaatcgtcc ctcaggcgtg     180
   agcaaccgtt ttagcggatc caaaagcggc aacaccgcga gcctgaccat tagcggcctg     240
   caagcggaag acgaagcgga ttattattgc caggcttggg attctaattt taagaatcgt     300
   gtgtttggcg gcggcacgaa gttaaccgtt cttggccagc cgaaagccgc accgagtgtg     360
   acgctgtttc cgccgagcag cgaagaattg caggcgaaca aagcgaccct ggtgtgcctg     420
   attagcgact tttatccggg agccgtgaca gtggcctgga aggcagatag cagccccgtc     480
   aaggcgggag tggagaccac cacaccctcc aaacaaagca acaacaagta cgcggccagc     540
   agctatctga gcctgacgcc tgagcagtgg aagtcccaca gaagctacag ctgccaggtc     600
   acgcatgagg ggagcaccgt ggaaaaaacc gttgcgccga ctgaggcctg ataa           654
<210> 25
   <211> 216
   <212> PRT
   <213> Artificial
<220>
   <223> Fab fragment MOR03268 VL-CL
<400> 25
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Arg Ser Trp Asp Ser Asn
                   85                  90                  95
   Leu Ser Tyr Ser Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 26
   <211> 654
   <212> DNA
   <213> Artificial
<220>
   <223> DNA coding for Fab fragment MOR03268 VL-CL
<400> 26
   gatatcgcac tgacccagcc agcttcagtg agcggctcac caggtcagag cattaccatc     60
   tcgtgtacgg gtactagcag cgatgttggt tctaataatt atgtgtcttg gtaccagcag    120
   catcccggga aggcgccgaa acttatgatt tatggtggtt ctaatcgtcc ctcaggcgtg    180
   agcaaccgtt ttagcggatc caaaagcggc aacaccgcga gcctgaccat tagcggcctg    240
   caagcggaag acgaagcgga ttattattgc cgttcttggg attctaatct ttcttattct    300
   gtgtttggcg gcggcacgaa gttaaccgtt cttggccagc cgaaagccgc accgagtgtg    360
   acgctgtttc cgccgagcag cgaagaattg caggcgaaca aagcgaccct ggtgtgcctg    420
   attagcgact tttatccggg agccgtgaca gtggcctgga aggcagatag cagccccgtc    480
   aaggcgggag tggagaccac cacaccctcc aaacaaagca acaacaagta cgcggccagc    540
   agctatctga gcctgacgcc tgagcagtgg aagtcccaca gaagctacag ctgccaggtc    600
   acgcatgagg ggagcaccgt ggaaaaaacc gttgcgccga ctgaggcctg ataa          654
<210> 27
   <211> 216
   <212> PRT
   <213> Artificial
<220>
   <223> Fab fragment MOR03292 VL-CL
<400> 27
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Ala Pro Leu
                   85                  90                  95
   Phe Lys Met Arg Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 28
   <211> 654
   <212> DNA
   <213> Artificial
<220>
   <223> DNA coding for Fab fragment MOR03292 VL-CL
<400> 28
   gatatcgcac tgacccagcc agcttcagtg agcggctcac caggtcagag cattaccatc     60
   tcgtgtacgg gtactagcag cgatgttggt tctaataatt atgtgtcttg gtaccagcag    120
   catcccggga aggcgccgaa acttatgatt tatggtggtt ctaatcgtcc ctcaggcgtg    180
   agcaaccgtt ttagcggatc caaaagcggc aacaccgcga gcctgaccat tagcggcctg    240
   caagcggaag acgaagcgga ttattattgc cagtcttggg ctcctctttt taagatgcgt    300
   gtgtttggcg gcggcacgaa gttaaccgtt cttggccagc cgaaagccgc accgagtgtg    360
   acgctgtttc cgccgagcag cgaagaattg caggcgaaca aagcgaccct ggtgtgcctg    420
   attagcgact tttatccggg agccgtgaca gtggcctgga aggcagatag cagccccgtc    480
   aaggcgggag tggagaccac cacaccctcc aaacaaagca acaacaagta cgcggccagc    540
   agctatctga gcctgacgcc tgagcagtgg aagtcccaca gaagctacag ctgccaggtc    600
   acgcatgagg ggagcaccgt ggaaaaaacc gttgcgccga ctgaggcctg ataa          654
<210> 29
   <211> 216
   <212> PRT
   <213> Artificial
<220>
   <223> Fab fragment MOR03294 VL-CL
<400> 29
   Asp Ile Ala Leu Thr Gln Pro Ala Ser Val Ser Gly Ser Pro Gly Gln
   1 5 10 15
   Ser Ile Thr Ile Ser Cys Thr Gly Thr Ser Ser Asp Val Gly Ser Asn
   20 25 30
   Asn Tyr Val Ser Trp Tyr Gln Gln His Pro Gly Lys Ala Pro Lys Leu
   35 40 45
   Met Ile Tyr Gly Gly Ser Asn Arg Pro Ser Gly Val Ser Asn Arg Phe
   50 55 60
   Ser Gly Ser Lys Ser Gly Asn Thr Ala Ser Leu Thr Ile Ser Gly Leu
   65 70 75 80
   Gln Ala Glu Asp Glu Ala Asp Tyr Tyr Cys Gln Thr Trp Thr Ser Ser
   85 90 95
   Phe Ser Ser Arg Val Phe Gly Gly Gly Thr Lys Leu Thr Val Leu Gly
   100 105 110
   Gln Pro Lys Ala Ala Pro Ser Val Thr Leu Phe Pro Pro Ser Ser Glu
   115 120 125
   Glu Leu Gln Ala Asn Lys Ala Thr Leu Val Cys Leu Ile Ser Asp Phe
   130 135 140
   Tyr Pro Gly Ala Val Thr Val Ala Trp Lys Ala Asp Ser Ser Pro Val
   145 150 155 160
   Lys Ala Gly Val Glu Thr Thr Thr Pro Ser Lys Gln Ser Asn Asn Lys
   165 170 175
   Tyr Ala Ala Ser Ser Tyr Leu Ser Leu Thr Pro Glu Gln Trp Lys Ser
   180 185 190
   His Arg Ser Tyr Ser Cys Gln Val Thr His Glu Gly Ser Thr Val Glu
   195 200 205
   Lys Thr Val Ala Pro Thr Glu Ala
   210 215
<210> 30
   <211> 654
   <212> DNA
   <213> Artificial
<220>
<223> DNA coding for Fab fragment MOR03294 VL-CL
<400> 30
   gatatcgcac tgacccagcc agcttcagtg agcggctcac caggtcagag cattaccatc      60
   tcgtgtacgg gtactagcag cgatgttggt tctaataatt atgtgtcttg gtaccagcag     120
   catcccggga aggcgccgaa acttatgatt tatggtggtt ctaatcgtcc ctcaggcgtg     180
   agcaaccgtt ttagcggatc caaaagcggc aacaccgcga gcctgaccat tagcggcctg     240
   caagcggaag acgaagcgga ttattattgc cagacttgga cttcttcttt ttcttctcgt     300
   gtgtttggcg gcggcacgaa gttaaccgtt cttggccagc cgaaagccgc accgagtgtg     360
   acgctgtttc cgccgagcag cgaagaattg caggcgaaca aagcgaccct ggtgtgcctg     420
   attagcgact tttatccggg agccgtgaca gtggcctgga aggcagatag cagccccgtc     480
   aaggcgggag tggagaccac cacaccctcc aaacaaagca acaacaagta cgcggccagc     540
   agctatctga gcctgacgcc tgagcagtgg aagtcccaca gaagctacag ctgccaggtc     600
   acgcatgagg ggagcaccgt ggaaaaaacc gttgcgccga ctgaggcctg ataa           654
<210> 31
   <211> 216
   <212> PRT
   <213> Artificial
<220>
   <223> Fab fragment MOR03295 VL-CL
<400> 31
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Ser Trp Asp Ser Ala
                   85                  90                  95
   Leu Ser Asn Arg Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 32
   <211> 654
   <212> DNA
   <213> Artificial
<220>
   <223> DNA coding for Fab fragment MOR03295 VL-CL
<400> 32
   gatatcgcac tgacccagcc agcttcagtg agcggctcac caggtcagag cattaccatc    60
   tcgtgtacgg gtactagcag cgatgttggt tctaataatt atgtgtcttg gtaccagcag   120
   catcccggga aggcgccgaa acttatgatt tatggtggtt ctaatcgtcc ctcaggcgtg   180
   agcaaccgtt ttagcggatc caaaagcggc aacaccgcga gcctgaccat tagcggcctg   240
   caagcggaag acgaagcgga ttattattgc cagtcttggg attctgctct ttctaatcgt   300
   gtgtttggcg gcggcacgaa gttaaccgtt cttggccagc cgaaagccgc accgagtgtg   360
   acgctgtttc cgccgagcag cgaagaattg caggcgaaca aagcgaccct ggtgtgcctg   420
   attagcgact tttatccggg agccgtgaca gtggcctgga aggcagatag cagccccgtc   480
   aaggcgggag tggagaccac cacaccctcc aaacaaagca acaacaagta cgcggccagc   540
   agctatctga gcctgacgcc tgagcagtgg aagtcccaca gaagctacag ctgccaggtc   600
   acgcatgagg ggagcaccgt ggaaaaaacc gttgcgccga ctgaggcctg ataa         654
<210> 33
   <211> 216
<212> PRT
   <213> Artificial
<220>
   <223> Fab fragment MOR03309 VL-CL
<400> 33
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Thr Trp Asp His Gly
                   85                  90                  95
   Phe Thr His Arg Val Phe Gly Gly Gly Thr Lys Leu Thr Val Leu Gly
               100                 105                 110
   Gln Pro Lys Ala Ala Pro Ser Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Ser Ser Pro Val
   145                 150                 155                 160
   Lys Ala Gly Val Glu Thr Thr Thr Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Ala
       210                 215
<210> 34
   <211> 654
   <212> DNA
   <213> Artificial
<220>
   <223> DNA coding for Fab fragment MOR03309 VL-CL
<400> 34
   gatatcgcac tgacccagcc agcttcagtg agcggctcac caggtcagag cattaccatc     60
   tcgtgtacgg gtactagcag cgatgttggt tctaataatt atgtgtcttg gtaccagcag    120
   catcccggga aggcgccgaa acttatgatt tatggtggtt ctaatcgtcc ctcaggcgtg    180
   agcaaccgtt ttagcggatc caaaagcggc aacaccgcga gcctgaccat tagcggcctg    240
   caagcggaag acgaagcgga ttattattgc cagacttggg atcatggttt tactcatcgt    300
   gtgtttggcg gcggcacgaa gttaaccgtt cttggccagc cgaaagccgc accgagtgtg    360
   acgctgtttc cgccgagcag cgaagaattg caggcgaaca aagcgaccct ggtgtgcctg    420
   attagcgact tttatccggg agccgtgaca gtggcctgga aggcagatag cagccccgtc    480
   aaggcgggag tggagaccac cacaccctcc aaacaaagca acaacaagta cgcggccagc    540
   agctatctga gcctgacgcc tgagcagtgg aagtcccaca gaagctacag ctgccaggtc    600
   acgcatgagg ggagcaccgt ggaaaaaacc gttgcgccga ctgaggcctg ataa          654
<210> 35
   <211> 213
   <212> PRT
   <213> Artificial
<220>
   <223> Fab fragment MOR03291 VL-CL
<400> 35
   Asp Ile Glu Leu Thr Gln Pro Pro Ser Val Ser Val Ala Pro Gly Gln
   1               5                   10                  15
   Thr Ala Arg Ile Ser Cys Ser Gly Asp Ser Ile Arg Ser Lys Tyr Val
               20                  25                  30
   His Trp Tyr Gln Gln Lys Pro Gly Gln Ala Pro Val Leu Val Ile Tyr
           35                  40                  45
   Arg Asp Asn Asn Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Glu
   65                  70                  75                  80
   Asp Glu Ala Asp Tyr Tyr Cys Ala Ser Tyr Asp Tyr Lys Ser Lys Asn
                   85                  90                  95
   Ile Val Phe Gly Gly Gly Thr Lys Leu Thr Val Leu Gly Gln Pro Lys
               100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
       130                 135                 140
   Ala Val Thr Val Ala Trp Lys Ala Asp Ser Ser Pro Val Lys Ala Gly
   145                 150                 155                 160
   Val Glu Thr Thr Thr Pro Ser Lys Gln Ser Asn Asn Lys Tyr Ala Ala
                   165                 170                 175
   Ser Ser Tyr Leu Ser Leu Thr Pro Glu Gln Trp Lys Ser His Arg Ser
               180                 185                 190
   Tyr Ser Cys Gln Val Thr His Glu Gly Ser Thr Val Glu Lys Thr Val
           195                 200                 205
   Ala Pro Thr Glu Ala
       210
<210> 36
   <211> 645
   <212> DNA
   <213> Artificial
<220>
   <223> DNA coding for Fab fragment MOR03291 VL-CL
<400> 36
   gatatcgaac tgacccagcc gccttcagtg agcgttgcac caggtcagac cgcgcgtatc    60
   tcgtgtagcg gcgattctat tcgttctaag tatgttcatt ggtaccagca gaaacccggg   120
   caggcgccag ttcttgtgat ttatcgtgat aataatcgtc cctcaggcat cccggaacgc   180
   tttagcggat ccaacagcgg caacaccgcg accctgacca ttagcggcac tcaggcggaa   240
   gacgaagcgg attattattg cgcttcttat gattataagt ctaagaatat tgtgtttggc   300
   ggcggcacga agttaaccgt tcttggccag ccgaaagccg caccgagtgt gacgctgttt   360
   ccgccgagca gcgaagaatt gcaggcgaac aaagcgaccc tggtgtgcct gattagcgac   420
   ttttatccgg gagccgtgac agtggcctgg aaggcagata gcagccccgt caaggcggga   480
   gtggagacca ccacaccctc caaacaaagc aacaacaagt acgcggccag cagctatctg   540
   agcctgacgc ctgagcagtg gaagtcccac agaagctaca gctgccaggt cacgcatgag   600
   gggagcaccg tggaaaaaac cgttgcgccg actgaggcct gataa                   645
<210> 37
   <211> 214
   <212> PRT
   <213> Artificial
<220>
   <223> Fab fragment MOR03285 VL-CL
<400> 37
   Asp Ile Val Leu Thr Gln Pro Pro Ser Val Ser Gly Ala Pro Gly Gln
   1               5                   10                  15
   Arg Val Thr Ile Ser Cys Ser Gly Ser Ser Ser Asn Ile Gly Ser Tyr
               20                  25                  30
   Tyr Val Tyr Trp Tyr Gln Gln Leu Pro Gly Thr Ala Pro Lys Leu Leu
           35                  40                  45
   Ile Tyr Gly Asn Ser Gln Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Ala Trp Thr Gly Ser Tyr
                   85                  90                  95
   Ala Thr Val Phe Gly Gly Gly Thr Lys Leu Thr Val Leu Gly Gln Pro
               100                 105                 110
   Lys Ala Ala Pro Ser Val Thr Leu Phe Pro Pro Ser Ser Glu Glu Leu
           115                 120                 125
   Gln Ala Asn Lys Ala Thr Leu Val Cys Leu Ile Ser Asp Phe Tyr Pro
       130                 135                 140
   Gly Ala Val Thr Val Ala Trp Lys Ala Asp Ser Ser Pro Val Lys Ala
   145                 150                 155                 160
   Gly Val Glu Thr Thr Thr Pro Ser Lys Gln Ser Asn Asn Lys Tyr Ala
                   165                 170                 175
   Ala Ser Ser Tyr Leu Ser Leu Thr Pro Glu Gln Trp Lys Ser His Arg
               180                 185                 190
   Ser Tyr Ser Cys Gln Val Thr His Glu Gly Ser Thr Val Glu Lys Thr
           195                 200                 205
   Val Ala Pro Thr Glu Ala
       210
<210> 38
   <211> 648
   <212> DNA
   <213> Artificial
<220>
   <223> DNA coding for Fab fragment MOR03285 VL-CL
<400> 38
   gatatcgtgc tgacccagcc gccttcagtg agtggcgcac caggtcagcg tgtgaccatc      60
   tcgtgtagcg gcagcagcag caacattggt tcttattatg tgtattggta ccagcagttg     120
   cccgggacgg cgccgaaact tctgatttat ggtaattctc agcgtccctc aggcgtgccg     180
   gatcgtttta gcggatccaa aagcggcacc agcgcgagcc ttgcgattac gggcctgcaa     240
   agcgaagacg aagcggatta ttattgccag gcttggactg gttcttatgc tactgtgttt     300
   ggcggcggca cgaagttaac cgttcttggc cagccgaaag ccgcaccgag tgtgacgctg     360
   tttccgccga gcagcgaaga attgcaggcg aacaaagcga ccctggtgtg cctgattagc     420
   gacttttatc cgggagccgt gacagtggcc tggaaggcag atagcagccc cgtcaaggcg     480
   ggagtggaga ccaccacacc ctccaaacaa agcaacaaca agtacgcggc cagcagctat     540
   ctgagcctga cgcctgagca gtggaagtcc cacagaagct acagctgcca ggtcacgcat     600
   gaggggagca ccgtggaaaa aaccgttgcg ccgactgagg cctgataa                  648
<210> 39
   <211> 216
   <212> PRT
   <213> Artificial
<220>
   <223> Fab fragment MOR03293 VL-CL
<400> 39
   Asp Ile Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Ser Asn
               20                  25                  30
   Asn Tyr Val Ser Trp Tyr Gln Gln His Pro Gly Lys Ala Pro Lys Leu
           35                  40                  45
   Met Ile Tyr Gly Gly Ser Asn Arg Pro Ser Gly Val Ser Asn Arg Phe
   50 55 60
   Ser Gly Ser Lys Ser Gly Asn Thr Ala Ser Leu Thr Ile Ser Gly Leu
   65 70 75 80
   Gln Ala Glu Asp Glu Ala Asp Tyr Tyr Cys Ser Ser Trp Thr Thr Ile
   85 90 95
   Tyr Arg Asn Arg Val Phe Gly Gly Gly Thr Lys Leu Thr Val Leu Gly
   100 105 110
   Gln Pro Lys Ala Ala Pro Ser Val Thr Leu Phe Pro Pro Ser Ser Glu
   115 120 125
   Glu Leu Gln Ala Asn Lys Ala Thr Leu Val Cys Leu Ile Ser Asp Phe
   130 135 140
   Tyr Pro Gly Ala Val Thr Val Ala Trp Lys Ala Asp Ser Ser Pro Val
   145 150 155 160
   Lys Ala Gly Val Glu Thr Thr Thr Pro Ser Lys Gln Ser Asn Asn Lys
   165 170 175
   Tyr Ala Ala Ser Ser Tyr Leu Ser Leu Thr Pro Glu Gln Trp Lys Ser
   180 185 190
   His Arg Ser Tyr Ser Cys Gln Val Thr His Glu Gly Ser Thr Val Glu
   195 200 205
   Lys Thr Val Ala Pro Thr Glu Ala
   210 215
<210> 40
   <211> 654
   <212> DNA
   <213> Artificial
<220>
   <223> DNA coding for Fab fragment MOR03293 VL-CL
<400> 40
   gatatcgcac tgacccagcc agcttcagtg agcggctcac caggtcagag cattaccatc   60
   tcgtgtacgg gtactagcag cgatgttggt tctaataatt atgtgtcttg gtaccagcag  120
   catcccggga aggcgccgaa acttatgatt tatggtggtt ctaatcgtcc ctcaggcgtg  180
   agcaaccgtt ttagcggatc caaaagcggc aacaccgcga gcctgaccat tagcggcctg  240
   caagcggaag acgaagcgga ttattattgc tcttcttgga ctactattta tcgtaatcgt  300
   gtgtttggcg gcggcacgaa gttaaccgtt cttggccagc cgaaagccgc accgagtgtg  360
   acgctgtttc cgccgagcag cgaagaattg caggcgaaca aagcgaccct ggtgtgcctg  420
   attagcgact tttatccggg agccgtgaca gtggcctgga aggcagatag cagccccgtc  480
   aaggcgggag tggagaccac cacaccctcc aaacaaagca acaacaagta cgcggccagc  540
   agctatctga gcctgacgcc tgagcagtgg aagtcccaca gaagctacag ctgccaggtc  600
   acgcatgagg ggagcaccgt ggaaaaaacc gttgcgccga ctgaggcctg ataa        654

## Beschreibung

Die vorliegende Offenbarung betrifft Emitter-bindende Peptide, die bei einer Wechselwirkung ihrer Antigen-Bindungstasche mit dem Emitter eine Veränderung der spektralen Emissionseigenschaften des Emitters bewirken. Die Emitter-bindende Peptide der Erfindung sind Bestandteile von Antikörper und Antikörperfragmenten.

### Hintergrund der Erfindung

Für den diagnostischen Nachhweis von Substanzen und deren Konzentrationsbestimmung werden heute in vielen Fällen *in-vitro* diagnostische Meßverfahren angewendet, die auf biologischen Molekülen, wie z. B. Peptiden, Proteinen, Antikörpern oder Oligonukleotiden, beruhen, die eine hohe Affinität für eine zu bestimmende Substanz haben. Bevorzugt werden hierfür Proteine und Peptide und besonders bevorzugt Antikörper und Antikörperfragmente verwendet.

Bestimmte in-vitro diagnostische Verfahren, wie z.B. die Elektrochemiluminienz basieren auf der Kombination verschiedener Antikörper gegen die zu bestimmende Substanz, wobei der eine Antikörper für die Abtrennung der zu bestimmenden Substanz aus der Untersuchungsprobe dient und der andere Antikörper das diagnostisch nachgewiesene Signalmolekül trägt. Im Falle des diagnostischen Verfahrens der Elektrochemolumineszenz wird der markierte Antikörper optisch detektiert [Grayeski, M. L., Anal. Chem. 1987, 59, 1243].

Neben der Elektrolumineszenz kann auch die lichtinduzierte Phosphoreszenz und die Fluoreszenz als optische Eigenschaft von Molekülen für diagnostische Meßverfahren verwendet werden. Gegenüber der Elektrolumineszenz und Phosphoreszenz bietet insbesondere die Fluoreszenz als optische Eigenschaft von Molekülen den Vorteil der hohen Nachweisempfindlichkeit und eine hohe Linearität des Meßsignals über einen großen dynamischen Bereich hinweg.

Zum Nachweis der Fluoreszenz eines Fluorophors wurden verschiedene Meßverfahren entwickelt, die unterschiedliche Prinzipien innerhalb der Fluoreszenzprozesse ausnutzen. Etablierte Meßverfahren nutzen z. B. die Abschwächung polarisierten Lichtes (Fluoreszenzpolarisierung - FP), die Messung der Photonenlebensdauer (Fluoreszenzlebensdauermessung - FLM), die Ausbleicheigenschaften (Fluoreszence-Photobleaching Recovery - FPR) und den Energietransfer zwischen verschiedenen Fluorophoren (Fluoreszenz-Resonanz-EnergieTransfer - FRET) [Williams, A. T., et al., Methods Immunol. Anal. 1993, 1, 466;Youn, H. J. et al., Anal. Biochem. 1995 Oswald, B. et al., Anal. Biochem. 2000, 280, 272; Szollosi, J. et. al., Cytometry 1998, 34, 159].

Andere Nachweisverfahren basieren auf einer Veränderung der Polarisationsebene oder dem Nachweis einer Phosphoreszenz.

Bei den oben genannten Verfahren erfüllen die verwendeten Anti-Substanz-Antikörper unterschiedliche Zwecke. Einerseits werden sie für die Abtrennung der zu bestimmenden Substanz aus der Probe verwendet, andererseits erfüllen sie aber auch die Aufgabe der Lokalisierung bzw. Positionierung unterschiedlicher verwendeter Signalgeber an der zu untersuchenden Substanz. Zum Nachweis z.B. eines Antikörpers in einer Probe haben sich vor allem optische und radioaktive Meßverfahren etabliert, aber auch akustische [siehe z.B. Cooper MA, et al. Direct and sensitive detection of a human virus by rupture event scanning. Nat Biotechnol. 2001 Sep;19(9):833-7.] und magnetische Meßverfahren sind bekannt. Die größte Verbreitung haben die optischen Meßverfahren erlangt [Nakamura, R. M., Dito, W. R., Tucker, E. S. (Eds.). Immunoassays: Clinical Laboratory Techniques for the 1980s. A. R. Liss, New York. Edwards, R. (ed.). Immunoassays: Essential Data, 1996, Wiley Europe.].

Bei der Mehrzahl der bereits verfügbaren Meßverfahren wird der Anti-Substanz-Antikörper mit einem Fluorophor markiert. Diese Markierung erfolgt durch spezifische und unspezifische chemische Kopplung. Der markierte Antikörper wird im Überschuß zu der Untersuchungsprobe zugegeben. Dies ist erforderlich, um alle zu untersuchenden Substanzmoleküle zu binden. Diesen Verfahren liegt weiterhin allgemein zu Grunde, daß der eine Anti-Substanz-Antikörper der Abtrennung der zu untersuchenden Substanz dient und der zweite Anti-Substanz-Antikörper, der an einer anderen Bindungsstelle der Untersuchungssubstanz erkennt, mit einem signalgebenden Molekül markiert ist. Auf diese Weise kann eine Verfälschung des Messergebnisses durch den nicht gebundenen, aber signalgebenden Antikörper vermieden werden. Diese Verfahrensweise ist jedoch, bedingt durch den Abtrennungsschritt, mit einem erhöhten methodischen und technischen Aufwand und höheren Kosten verbunden. Besonders nachteilig erweist sich jedoch der hohe technische Aufwand, der eine Etablierung dieses Verfahrens für die Schnelldiagnostik verhindert.

Antikörper und Peptide, die gegen Moleküle kleinen Molekulargewichts gerichtet sind, sind bereits bekannt. Darunter fallen auch Antikörper und Peptide gegen Farbstoffmoleküle. So beschreiben Simeonov A. et al. in Science 2000, 290, 307-313 Antikörper gegen Stilbene ("Blue-fluorescent antibodies"). Diese Antikörper katalysieren spezifisch photochemische Isomerierungsvorgänge und führen zu rotverschobenen Absorptions- und Fluoreszenzmaxima im UV-VIS Spektralbereich (Absorptionsverschiebung maximal 12 nm, Fluoreszenzverschiebung 22 nm). Simeonov et al. geben jedoch keinen Hinweis auf eine Rotverschiebung unter Beibehalt der Fluoreszenzquantenausbeute bei Cyaninfarbstoffen im Wellenlängenbereich von 600-1200 nm.

Watt R. M. et al. (Immunochemistry 1977, 14, 533-541) beschreiben die spektralen Eigenschaften von bereits bekannten anti-Fluorescein-Antikörperkonstrukten. Der Antikörper bewirkt nach Bindung des Fluoresceins eine Verschiebung des Absorptions- und Fluoreszenzmaximums im sichtbaren Spektralbereich, jedoch nur um 12 nm bzw. 5 nm. Zusätzlich erfolgt eine starke Verringerung der Fluoreszenzquantenausbeute um ca. 90%.

Rozinov M. N. et al. (Chem. Biol. 1998, 5, 713-728) beschreiben die Selektion 12-merer Peptide aus Phagenbibliotheken, die die Farbstoffe Texas Red, Rhodamine Red, Oregon Green 514 und Fluorescein binden. Für Texas Red wurde eine Rotverschiebung der Absorption und Fluoreszenz beobachtet, jedoch nur um 2.8 nm bzw. 1.4 nm.

Des weiteren sind Antikörper gegen verschiedene Farbstoffe bereits kommerziell erhältlich, z. B. gegen Fluorescein, Tetramethylrhodamin, Texas Red, Alexa Fluor 488, BODIPY FL, Lucifer yellow and Cascade Blue, Oregon Green (Fa. Molecular Probes, Inc., USA). Hierbei handelt es sich jedoch um polyklonale IgG Antikörper für bioanalytische Zwecke, die zum Teil unkontrollierbare Kreuzreaktivitäten aufweisen und nicht aus einem strikten Selektionsprozess hervorgegangen sind.

Es besteht somit ein weiterer Bedarf an verbesserten Emitter-bindenden Peptiden und insbesondere spezifischen Antikörpern, die besser für die oben genannten Meßverfahren geeignet werden. Vorteilhaft wären dabei insbesondere Emitter-bindende Peptide, die eine Rotverschiebung unter Beibehalt der Fluoreszenzquantenausbeute bei Cyaninfarbstoffen im Wellenlängenbereich von 600-1200 nm bewirken würden.

Diese Aufgabe wird erfindungsgemäß durch das in den Ansprüchen definierte Emitter-bindendes Peptid gelöst, das dadurch gekennzeichnet ist, daß dieses bei einer Wechselwirkung seiner Antigenbindungstasche mit dem Emitter eine Veränderung der spektralen Emissionseigenschaften des Emitters gewirkt, einem Verfahren zur Herstellung eines Emitter-bindenden Peptids gemäß der Erfindung, umfassend die Immunisierung eines geeigneten Organismus mit einem Emitter, umfassend einen Farbstoff, der ausgewählt ist aus der Gruppe von Polymethinfarbstoffen, wie Dicarbocyanin-, Tricarbocyanin-, Indotricarbocyanin-, Merocyanin-, Styryl-, Squarilium- und Oxonolfarbstoffen und Rhodaminfarbstoffen, Phenoxazin- oder Phenothiazinfarbstoffen und entsprechenden Verwendungen eines Emitter-bindenden Peptids, einer Nukleinsäure, einer Wirtszelle oder eines Antikörpers oder Konjugats gemäß der Erfindung als Diagnostikum für die *in vitro* Diagnostik. Zweckmäßige Ausgestaltungen sind in den abhängigen Ansprüchen aufgeführt.

Ein erster Aspekt der vorliegenden Erfindung betrifft somit ein wie in Anspruch 1 definiertes Emitter-bindendes Peptid, dadurch gekennzeichnet, daß dieses bei einer Wechselwirkung seiner Antigenbindungstasche mit dem Emitter eine Veränderung der spektralen Emissionseigenschaften des Emitters bewirkt.

Der Emitter umfaßt einen Farbstoff der mindestens ein Absorptionsmaximum und/oder Fluoreszenzmaximum innerhalb des Spektralbereiches von 700 bis 1000 nm, bevorzugt mindestens ein Absorptionsmaximum und Fluoreszenzmaximum innerhalb des Spektralbereiches von 750 bis 900 nm aufweist.

Weiter bevorzugt ist ein erfindungsgemäßes Emitter-bindendes Peptid, wobei die Veränderung der Emissionseigenschaften des Teils des Emitters ausgewählt ist aus einer Veränderung der Polarisationsebene, der Fluoreszenzintensität, der Phosphoreszenzintensität, der Fluoreszenzlebensdauer und einer bathochromen Verschiebung des Absorptionsmaximums und/oder des Fluoreszenzmaximums. Die Erfindung ist jedoch nicht auf diese speziellen Phänomene beschränkt, der Begriff "Veränderung der Emissionseigenschaften" im Rahmen der vorliegenden Erfindung soll alle physikalischen Phänomene oder Effekte umfassen, bei dem die auf den Emitter treffende energiereiche Strahlung in ihrer Eigenschaft verändert wird und diese Veränderung dabei von der Bindung/nicht-Bindung des Substanz-Emitter-Konjugats oder Substanz-erkennendes Mittel-Emitter-Konjugat mit seinem Emitter-Bindungspartner und der Substanz quantitativ abhängig ist. Die Substanz ist ein Antikörper oder ein Antikörperfragment. Im Rahmen der vorliegenden Erfindung handelt es sich bei den Antikörperfragmenten um Fragmente, die zumindest die Antigen-bindenden Bereiche umfassen, die die sogenannten "complementarity-determining regions" ("CDRs") enthalten. Bevorzugterweise umfassen die Antigen-bindenden Bereiche dabei die vollständigen variablen Ketten VL und VH.

In einem besonders bevorzugten Aspekt des Emitter-bindenden Peptids gemäß der vorliegenden Erfindung ist der Antikörper oder das Antikörperfragment ausgewählt aus polyklonalen oder monoklonalen Antikörpern, humanisierten Antikörpern, Fab-Fragmenten, insbesondere monomeren Fab-Fragmenten, scFV-Fragmenten, synthetischen und rekombinanten Antikörpern, scTCR-Ketten und Gemischen davon.

Insbesondere bevorzugt sind dabei im Falle der synthetischen und rekombinanten Antikörper oder Antikörperfragmente diejenigen aus einer HuCAL-Bibliothek (WO 97/08320; Knapppik,(2000), J. Mol. Biol. 296, 57-86; Krebs et al. J Immunol Methods. 2001 Aug 1;254(1-2):67-84). Diese können entweder als vollständige Immunoglobuline oder Antikörper in einem der natürlich vorkommenden Formate (IgA, IgD, IgE, IgG, IgM) oder als Antikörper-fragmente vorliegen, wobei die Antikörperfragmente zumindest die Aminosäurepositionen 4 bis 103 für VL und 5 bis 109 für VH, bevorzugt die Aminosäurepositionen 3 bis 107 für VL und 4 bis 111 für VH und besonders bevorzugt die vollständigen variablen Ketten VL und VH (Aminosäurepositionen 1 bis 109 für VL und 1 bis 113 für VH) umfassen (Numerierung nach WO 97/08320).

In einer bevorzugten Ausführungsform umfaßt der Antikörper oder das Antikörperfragment dabei zumindest einen der in den Sequenzen SEQ-ID NOs: 1, 2, 5, 6, 9, 10, 13, 14, 17, 19, 21, 23, 25, 27, 29, 31, 33, 35, 37 und 39 enthaltenen CDR-Bereiche (VL: CDR1 Positionen 24-34, CDR2 Positionen 50-56, CDR3 Positionen 89-96; VH: CDR1 Positionen 26-35, CDR2 Positionen 50-65, CDR3 Positionen 95-102), insbesondere VL CDR3 oder VH CDR3. Besonders bevorzugt ist dabei ein Antikörper, der eine der in den Sequenzen SEQ-ID NOs: 2, 6, 10, 14, 17, 19, 21, 23, 25, 27, 29, 31, 33, 35, 37 und 39 enthaltenen variablen Ketten VL oder eine in den Sequenzen SEQ-ID NOs: 1, 5, 9 und 13 enthaltenen variablen Ketten VH umfaßt (oder ein Fragment eines solchen Antikörpers). Am meisten bevorzugt ist ein Antikörper, der eines VH/VL-Paare umfaßt, die in den folgenden Sequenzpaaren enthalten sind: SEQ-ID NOs: 1+2; SEQ-ID NOs: 5+6; SEQ-ID NOs: 9+10; SEQ-ID NOs: 13+14; SEQ-ID NOs: 5+17; SEQ-ID NOs: 5+19; SEQ-ID NOs: 5+37; SEQ-ID NOs: 9+21; SEQ-ID NOs: 9+23; SEQ-ID NOs: 9+25; SEQ-ID NOs: 9+27; SEQ-ID NOs: 9+29; SEQ-ID NOs: 9+31; SEQ-ID NOs: 9+33; SEQ-ID NOs: 9+39; SEQ-ID NOs: 13+35 (oder ein Fragment eines solchen Antikörpers). Insbesondere bevorzugt sind dabei die Fab-Fragmente MOR02628, MOR02965, MOR02977, MOR02969, MOR03263, MOR03325, MOR03285, MOR03201, MOR03267, MOR03268, MOR03292, MOR03294, MOR03295, MOR03309, MOR03293 und MOR03291. Ausgehend von den so beschriebenen erfindungsgemäßen Antikörpern ergeben sich für den Fachmann in naheliegender Weise verschiedene Möglichkeiten, neue modifizierte Antikörper zu erhalten, die die erfindungsgemäßen Eigenschaften ebenfalls zeigen.

Dem Fachmann ist bekannt, daß für die Affinität sowie Selektivität und Spezifität insbesondere die CDR-Bereiche sowohl der schweren als auch der leichten Kette verantwortlich sind. Dabei spielen insbesondere der CDR3-Bereich von VH und der CDR3-Bereich von VL ein Rolle, gefolgt von CDR2 von VH und CDR1 von VL, während CDR1 von VH und CDR2 von VL zumeist eine untergeordnete Rolle spielen. Zur Optimierung von Affinität sowie Selektivität und Spezifität von Antikörpern bieten sich daher insbesondere die CDR-Bereiche an (s. auch z. B. Schier et al., J. Mol. Biol. (1996) 263, 551). Dabei können, zum Beispiel ein oder mehrere der CDR-Bereiche ausgetauscht werden, zum Beispiel gezielt gegen CDR-Bereiche von anderen, die erfindungsgemäßen Eigenschaften bereits zeigenden Antikörpern, oder aber durch Bibliotheken von entsprechenden CDR-Sequenzen (siehe hierzu die Optimierung in Beispiel 1), die entweder vollständig zufällige Variationen erzeugen oder einen mehr oder weniger starke Bevorzugung (Tendenz) in Richtung von bestimmten Aminosäuren oder Kombinationen davon enthalten. Der Fachmann ist darüber hinaus auch in der Lage, ganze variable Ketten in gleicher Weise gegen entsprechende Ketten anderer definierter Antikörper oder gegen diverse Bibliotheken solcher Ketten auszutauschen. Des weiteren sind dem Fachmann Verfahren bekannt, gezielt durch Mutagenese eine oder mehrere Aminosäurereste in den CDRs auszutauschen. Die Identifikation derartig zu verändernder Aminosäurereste erfolgt dabei z. B. auf Basis eines Vergleichs der Sequenzen verschiedener Antikörper und die Identifikation von konservierten oder zumindest hoch homologen Resten an den entsprechenden Positionen. Bei den Veränderungen an den CDRs verfügt der Fachmann dabei auch über Kenntnisse zu den sogenannten "kanonischen Strukturen" (Al-Lazikani et al., J. Mol. Biol. (2000) 295, 979); Knappik et al. J. Mol. Biol. (2000) 296, 57), die einen Einfluß auf die dreidimensionale Anordnung der CDR-Bereiche haben und die beim Design entsprechender Optimierungsstrategien berücksichtigt werden können.

Darüber hinaus sind dem Fachmann Techniken vertraut, auch die Gerüstregionen von Antikörpern oder Antikörperfragmenten zu verändern, um zu stabileren oder besser exprimierbaren Molekülen zu gelangen (WO 92/01787; Nieba et al. (1997) Protein Eng. 10, 435; Ewert et al. (2003) Biochemistry 42, 1517).

Über diese bereits beschriebenen Strategien zur Modifikation von Antikörpern oder Antikörperfragmenten hinaus, ist der Fachmann in Kenntnis der erfindungsgemäßen Antikörper und der in der vorliegenden Anmeldung beschriebenen Meßverfahren auch in der Lage, weitere Veränderung an der Aminosäuresequenz oder der Zusammensetzung der beschriebenen Antikörper vorzunehmen und durch Anwendung der beschriebenen Assays und Meßverfahren zu entscheiden, ob modifizierte Antikörper entstanden sind, deren Eigenschaften mit denen übereinstimmen, die die erfindungsgemäßen Antikörper auszeichnen.

Ein weiterer Aspekt der vorliegenden Erfindung betrifft Nukleinsäuremoleküle, die einen der erfindungsgemäßen Antikörper oder ein Antikörperfragment kodieren. In einer bevorzugten Ausführungsform handelt es sich dabei um Nukleinsäuremoleküle, die eine der in den Sequenzen SEQ-ID NOs: 2, 6, 10, 14, 17, 19, 21, 23, 25, 27, 29, 31, 33, 35, 37 und 39 enthaltenen variablen Ketten VL oder eine in den Sequenzen SEQ-ID NOs: 1, 5, 9 und 13 enthaltenen variablen Ketten VH kodieren. Besonders bevorzugt sind dabei die Sequenzen gemäß der SEQ-ID NOs: 3, 4, 7, 8, 11, 12, 15, 16, 18, 20, 22, 24, 26, 28, 30, 32, 34, 36, 38 und 40.

Das Emitter-bindendes Peptid der vorliegenden Erfindung weist optimalerweise eine Bindungsaffinität von kleiner als 50 nM und bevorzugt von kleiner als 10 nM auf.

Ein weiterer Aspekt ist ein Emitter-bindendes Peptid der vorliegenden Erfindung, wobei der Emitter einen Farbstoff umfaßt, der mindestens ein Absorptionsmaximum und/oder Fluoreszenzmaximum innerhalb des Spektralbereiches von 700 bis 1000 nm, bevorzugt mindestens ein Absorptionsmaximum und Fluoreszenzmaximum innerhalb des Spektralbereiches von 750 bis 900 nm aufweist. Die bathochrome Verschiebung des Farbstoffs ist so gewählt, daß die Verschiebung des Absorptions- und/oder Fluoreszenzmaximums zu höheren Wellenlängen nach Wechselwirkung mit dem Mittel zur Erkennung des Emitters um einen Wert von größer 15 nm, bevorzugt größer 25 nm, und am meisten bevorzugt um ungefähr 30 nm erfolgt. Dabei muß nicht unbedingt eine Verschiebung als solche betrachtet werden, die eine Eigenschaft des Farbstoffs ist. Üblicherweise würde die Verschiebung als Veränderung eines an den Farbstoff angepaßten Emissionswertes gemessen werden, also bei einer bestimmten singulären Wellenlänge. Dafür sind geeignete optische Mitteln zur Messung vorgesehen, die dem Fachmann bekannt sind. Dies gilt ebenfalls für die Messung der Veränderung der Polarisationsebene, der Fluoreszenzintensität, der Phosphoreszenzintensität, der Fluoreszenzlebensdauer und einer bathochromen Verschiebung des Absorptionsmaximums und/oder des Fluoreszenzmaximums.

Für die erfindungsgemäßen Emitter-bindenden Peptide ist der verwendete Emitter einen Farbstoff, der ausgewählt ist aus der Gruppe von Polymethinfarbstoffen, wie Dicarbocyanin-, Tricarbocyanin-, Indotricarbocyanin-, Merocyanin-, Styryl-, Squarilium- und Oxonolfarbstoffen und Rhodaminfarbstoffen, Phenoxazin- oder Phenothiazinfarbstoffen. Allgemein kann der Emitter des erfindungsgemäßen Substanz-Emitter-Konjugats einen Cyaninfarbstoff der allgemeinen Formel (I) 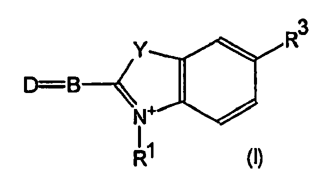 umfassen, in der D für einen Rest (II) oder (III) 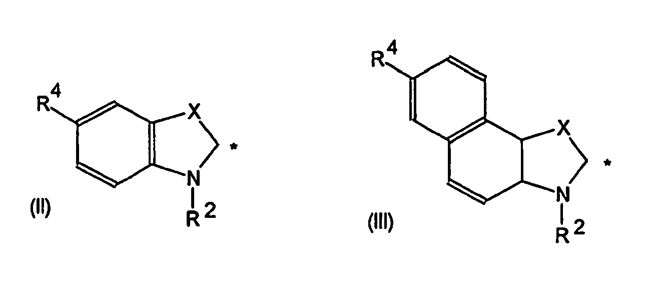 steht, wobei die mit dem Stern markierte Position die Verknüpfungsstelle mit dem Rest B bedeutet und B für die Gruppe (IV), (V), (VI), (VII) oder (VIII) 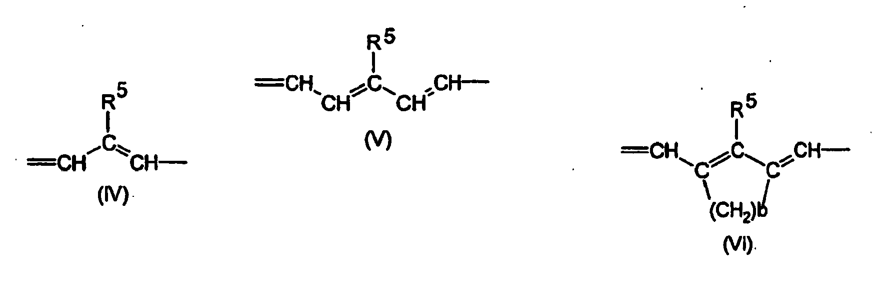 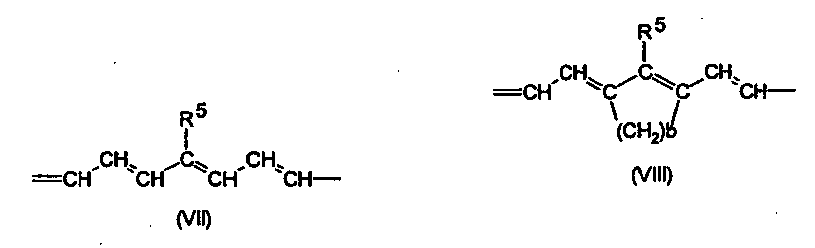 stehen kann, in denen R¹ und R² unabhängig voneinander eine C₁-C₄-Sulfoalkylkette, eine gesättigte oder ungesättigte, verzweigte oder geradekettige C₁-C₅₀-Alkylkette darstellt, die gegebenenfalls mit von 0 bis 15 Sauerstoffatomen und/oder von 0 bis 3 Carbonylgruppen unterbrochen ist und/oder mit 0 bis 5 Hydroxygruppen substituiert sein kann, bedeutet, R³ und R⁴ unabhängig voneinander für die Gruppe -COOE¹, -CONE¹E², NHCOE¹, -NHCONHE¹, - NE¹E², -OE¹, -OSO₃E¹, -SO₃E¹, -SO₂NHE¹ oder -E¹ stehen, wobei E¹ und E² unabhängig voneinander ein Wasserstoffatom, eine C₁-C₄-Sulfoalkylkette, eine gesättigte oder ungesättigte, verzweigte oder geradekettige C₁-C₅₀-Alkylkette darstellt, die gegebenenfalls von 0 bis 15 Sauerstoffatomen und/oder von 0 bis 3 Carbonylgruppen unterbrochen ist und/oder mit 0 bis 5 Hydroxygruppen substituiert ist, R⁵ für ein Wasserstoffatom, eine Methyl-, Ethyl- oder Propylgruppe oder ein Fluor- Chlor, Brom- oder Jodatom steht, b die Zahl 2 oder 3 bedeutet, und X und Y unabhängig voneinander für O, S, =C(CH₃)₂ oder-(CH=CH)- steht, sowie Salze und Solvate dieser Verbindungen.

Überraschenderweise konnte gefunden werden, daß nach hochaffiner Bindung eines Antikörpers an einen Cyaninfarbstoff mit Absorption und Fluoreszenz im Nahinfraroten Spektralbereich (> 750 nm) eine Verschiebung des Absorptionsmaximums und Fluoreszenzmaximums um ca. 30 nm zu höheren Wellenlängen erfolgte (bathochrome Verschiebung). Unter Ausnutzung dieses Prinzips ist es somit zum Beispiel möglich, über einen großen Konzentrationsbereich ein Signal aus einer Vollblutprobe direkt und spektral getrennt zu detektieren, wobei sich das Signal linear zur Konzentration der zu bestimmenden Substanz verhält.

Die Emitter können mit Substanzen Konjugate bilden. Im Rahmen der vorliegenden Erfindung werden als Substanz-Emitter-Konjugate solche der allg, Formel
S-E
verwendet, worin S für eine zu untersuchende Substanz und E für einen Emitter steht, der einen Teil umfaßt, der auf eine Wechselwirkung mit dem Mittel zur Erkennung des Emitters mit einer Veränderung der Emissionseigenschaften reagiert. Als struktureller Bestandteil der erfindungsgemäßen Konjugate eignen sich u.a. Farbstoffe, die mindestens ein Absorptionsmaximum und Fluoreszenzmaximum innerhalb des Spektralbereiches von 600 bis 1200 nm besitzen. Bevorzugt sind dabei Farbstoffe mit mindestens ein Absorptionsmaximum und Fluoreszenzmaximum innerhalb des Spektralbereiches von 700 bis 1000 nm besitzen. Farbstoffe, die diese Kriterien erfüllen, sind beispielsweise solche aus folgenden Klassen: Polymethinfarbstoffe, wie Dicarbocyanin-, Tricarbocyanin-, Merocyanin- und Oxonolfarbstoffe, Rhodaminfarbstoffe, Phenoxazin- oder Phenothiazinfarbstoffe, Tetrapyrrolfarbstoffe, insbesondere Benzoporphyrine, Chorine, Bacteriochlorine, Pheophorbide, Bacteriopheophorbide, Purpurine und Phthalocyanine.

Bevorzugte Farbstoffe sind die Cyaninfarbstoffe mit Absorptionsmaxima zwischen 750 und 900 nm, mit besonderem Vorteil Indotricarbocyanine. Struktureller Bestandteil der erfmdungsgemäßen Konjugate sind auch die Substanzen, deren Konzentrationsbestimmung mittels des erfindungsgemäßen Verfahrens erfolgen soll.

Diese sind beispielsweise ausgewählt aus Antigenen, wie Proteinen, Peptiden, Nukleinsäuren, Oligonukleotiden, Blutkomponenten, Serumkomponenten, Lipiden, Pharmaka und Verbindungen niederen Molekulargewichts, insbesondere Zuckern, Farbstoffen oder anderen Verbindungen mit einem Molekulargewicht von unter 500 Dalton.

Bevorzugte Farbstoffe sind die Cyaninfarbstoffe mit Absorptionsmaxima zwischen 750 und 900 nm, mit besonderem Vorteil Indotricarbocyanine.

Die Farbstoffe enthalten Strukturelemente, über welche die kovalente Kopplung an die Substanzstrukuren erfolgt. Dieses sind z. B. Linker mit Carboxygruppen, Aminogruppen, Hydroxygruppen.

Im Falle einer optischen Messung kann diese in unterschiedlicher Weise erfolgen und richtet sich hauptsächlich nach der Art der charakteristischen Veränderung der spektralen Eigenschaften des Emitters (z.B. Fluorophors). Generell bevorzugt ist eine Erfassung der Verschiebung der Absorptionswellenlänge und Emissionswellenlänge oder die Messung der Absorption und / oder Fluoreszenzintensität bei einer Wellenlänge, die zum größten Teil den am Antikörper gebundenen Anteil des Fluorophors erfaßt. Je nach Veränderung der spektralen Eigenschaften des antikörpergebundenen Fluorophors können auch andere Eigenschaften, wie z. B. die Photonenlebensdauer, die Polarisation und das Ausbleichverhalten zur optischen Messung verwendet werden.

Der besondere Vorteil von Fluorophoren im spektralen Bereich des nahinfraroten Lichtes liegt in der geringen Überdeckung durch Bestandteile des Blutes. Hierdurch wird eine Tiefeneindringung ermöglicht, ohne daß das zu detektierende Signal unverhältnismäßig stark verändert wird.

Dem Fachmann sind darüber hinaus auch bereits Antikörper gegen Fluorophore bekannt, die in der Lage sind, nach Bindung des Fluorophors dessen spektrale Eigenschaften im UV-Bereich zu verändern. Durch Bindung eines Fluorophors in die Antigenbindungstasche eines Antikörpers können vor allem die Fluoreszenzintensität, das Absorptionsmaximum, das Emissionsmaximum, und die Photonenlebensdauer verändert werden [Simeonov A., et al., Science (2000) 307-313]. Diese bekannten Antikörper sind jedoch gegen Emitter (Fluorophore) gerichtet, die ihre Absorption und Fluoreszenzemission im sichtbaren und UV-Bereich des Lichts haben.

Ein weiterer Aspekt der vorliegenden Erfindung betrifft die Verwendung eines erfindungsgemäßen Emitter-bindenden Peptids für die *in vitro* Diagnostik.

Zu diesem Zweck kann das erfindungsgemäße Emitter-bindende Peptid auch in einem diagnostischen Kit vorliegen, gegebenenfalls zusammen mit anderen Hilfsstoffen. Alle erfindungsgemäßen Kits können weiterhin spezielle Instruktionen und Unterlagen (z.B. Eichkurven, Anweisung zur Quantifizierung, usw.) enthalten.

Die Erfindung soll nun im folgenden anhand von Beispielen und den beigefügten Figuren und Sequenzen näher beschrieben werden, ohne jedoch darauf beschränkt zu sein. Es zeigen:
**Figur 1****:** Den CysDisplay-Screening Vektor pMORPH23 (Vektorkarte und Sequenz),
**Figur 2****:** Den Expressionsvektor pMORPHX9 MS (Vektorkarte und Sequenz), und

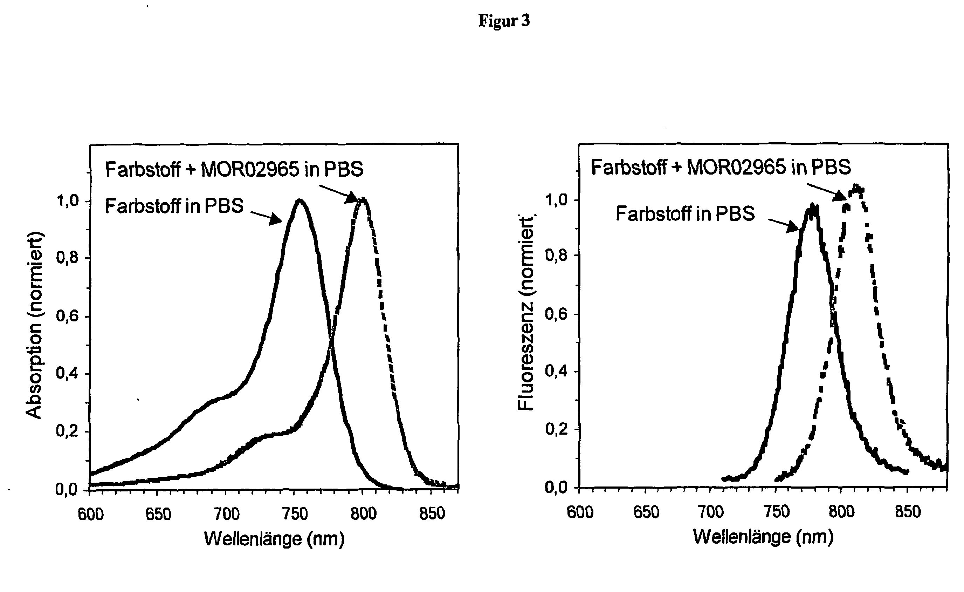

**Figur 3****:** Absorptionspektrum (links) und Fluoreszenzspektrum vom Farbstoff aus Beispiel 2 in Abwesenheit und in Gegenwart von Antikörper MOR02965 in PBS.

### Beispiele:

### Beispiel 1: Selektionierung, Herstellung und Charakterisierung von Emitter-bindenden Antikörpern: Selektion von HuCAL GOLD Fab Antikörper-Fragmenten gegen den Cyanin-Farbstoff Fuji 6-4 (ZK203468) [Trinatrinm-3,3-dimethyl-2-{4-methyl-7-[3,3-dimethyl-5-sulfonato-1-(2-sulfonatoethyl)-3H-indolium-2-yl]hepta-2,4,6-trien-1-yliden}-1-(2-sulfonatoethyl)-2,3-dihydro-1H-indol-5-sulfonat, inneres Salz]

### HuCAL GOLD Antikörperbibliothek:

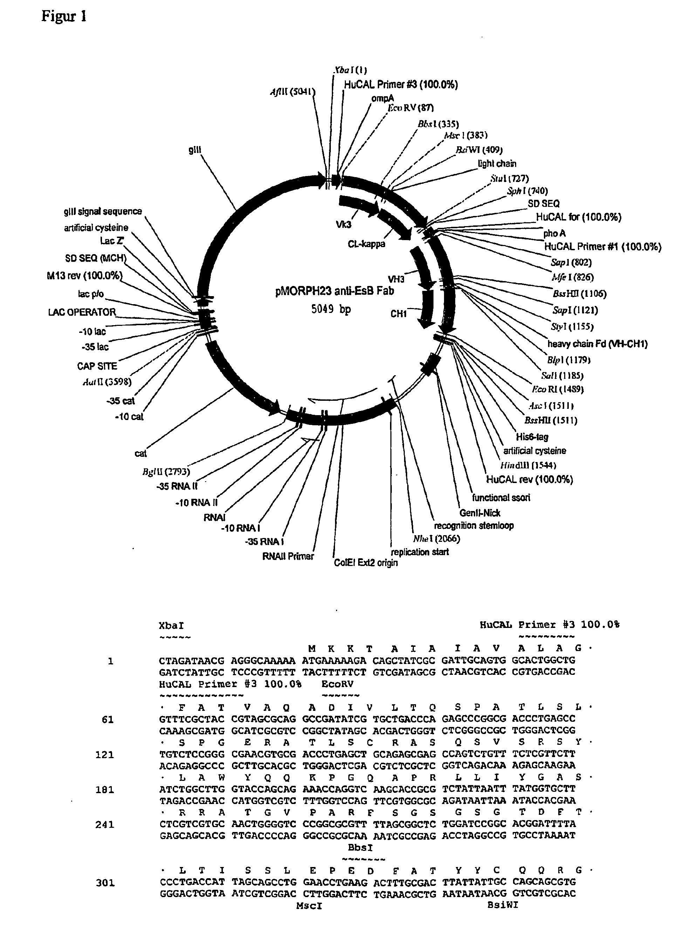

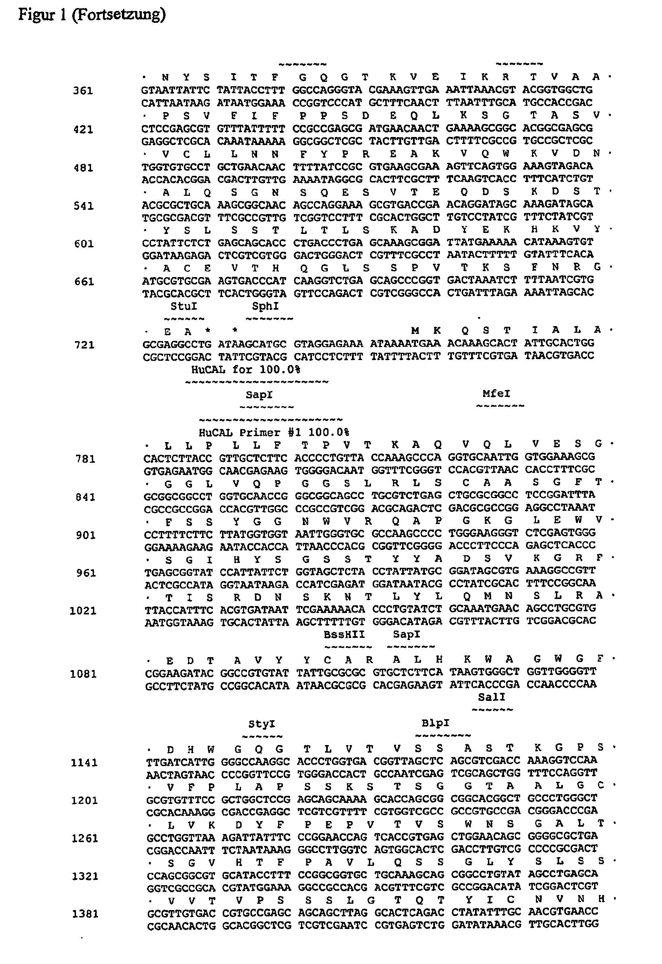

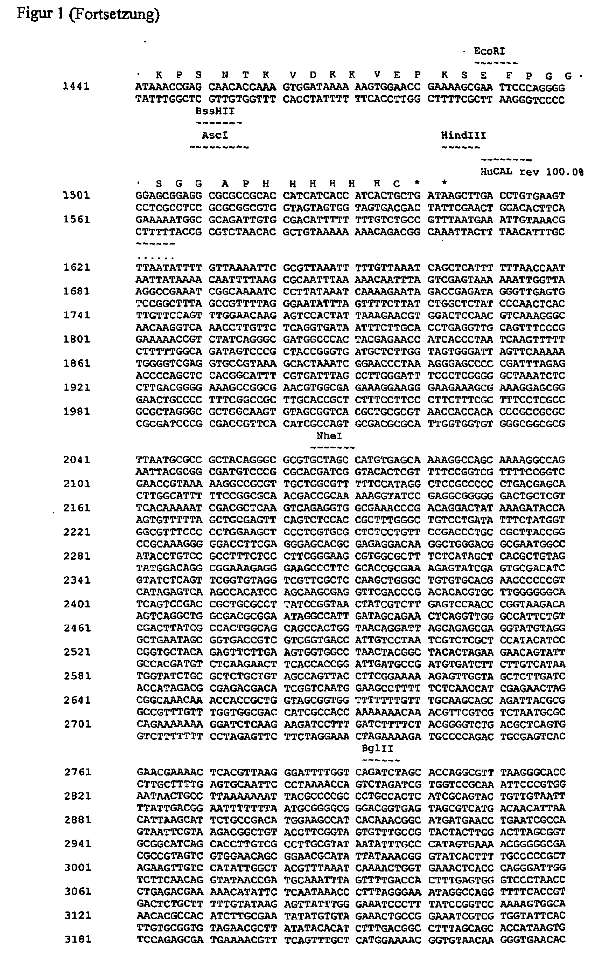

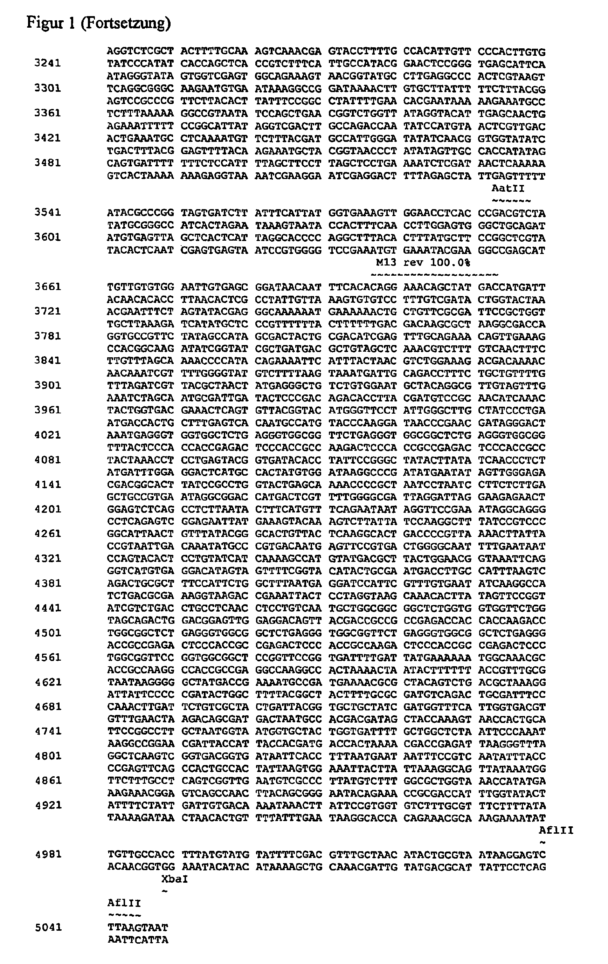

Antikörperbibliothek HuCAL GOLD: HuCAL GOLD ist eine vollsynthetische, modulare humane Antikörperbibliothek im Fab Antikörperfragmentformat. HuCAL GOLD basiert auf den HuCAL-Konsensus-Antikörpergenen, die für die Bibliothek HuCAL-scFv1 beschrieben wurden (WO 97/08320; Knappik,(2000), J. Mol. Biol. 296, 57-86; Krebs et al. J Immunol Methods. 2001 Aug 1;254(1-2):67-84). In HuCAL GOLD sind alle sechs CDR-Bereiche durch den Einsatz der sogenannten Trinukleotidmutagenese (Virnekäs et al. (1994) Nucleic Acids Res. 1994 Dec 25;22(25):5600-7) entsprechend der Zusammensetzung dieser Bereiche in menschlichen Antikörpern diversifiziert, während in früheren HuCAL-Bibliotheken (HuCAL-scFvl und HuCAL-Fab1 lediglich die CDR3-Bereiche in VH und VL entsprechend der natürlichen Zusammensetzung (s. Knappik et al., 2000) diversifiziert worden waren. Darüber hinaus findet in HuCAL GOLD auch ein abgewandeltes Screening-Verfahren Verwendung, das sogenannte CysDisplay (WO 01/05950). Der für das Screeningverfahren verwendete Vektor pMORPH23 findet sich in Figur 1.

*Vλ Positionen 1 and* 2. Die ursprünglichen HuCAL Mastergene wurden mit ihren authentischen N-Termini konstruiert: VLλ1: QS (CAGAGC), VLλ2: QS (CAGAGC), and VLλ3: SY (AGCTAT). Diese Sequenzen finden sich in WO 97/08320. Bei der Herstellung der HuCAL-scFv1-Bibliothek wurden diese beiden Aminosäurereste in "DI" geändert, um das Klonieren zu erleichtern (*EcoRI* site). Diese Reste wurden bei der Herstellung von HuCAL-Fab1 und HuCAL GOLD beibehalten. Daher enthalten alle HuCAL-Bibliotheken VLλ-Gene mit der*EcoRV*-Schnittstelle GATATC (DI) am 5'-Ende. Alle HuCAL kappa Gene (Mastergene und alle Gene in den Bibliotheken) enthalten ohnehin DI am 5'-Ende, da dies die authentischen N-Termini darstellen (WO 97/08320).

*VH Position 1.* Die ursprünglichen HuCAL-Mastergene wurden mit ihren authentischen N-termini hergestellt: VH1A, VH1B, VH2, VH4, und VH6 mit Q (=CAG) als erstem Aminosäurerest und VH3 sowie VH5 mit E (=GAA). Die entsprechenden Sequenzen finden sich in WO 97/08320. Bei der Klonierung der HuCAL-Fab1 sowie der HuCAL GOLD Bibliothek wurde an dieser Position 1 die Amiosäure Q (CAG) in allen VH-Genen eingebaut.

### Phagemid-Produktion

Durch Infektion von *E. coli* TOP10F'-Zellen aus der HuCAL GOLD Antikörper-Bibliothek bzw. aus den Maturierungsbibliotheken mittels Helferphagen wurden große Mengen an Phagemiden produziert und angereichert. Dazu wurden die HuCAL GOLD bzw. die Maturierungsbibliotheken (in den TOP10F'-Zellen) in 2xYT-Medium mit 34 µg/ml Chloramphenicol/ 10 µg/ml Tetrazyklin/ 1% Glucose bei 37°C bis zu einer OD₆₀₀ von 0.5 kultiviert. Anschließend erfolgte die Infektion mit VCSM13 Helferphagen bei 37°C. Die infizierten Zellen wurden pelletiert und in 2xYT/ 34 µg/ml Chloramphenicol/ 10 µg/ml Tetrazyklin/ 50 µg/ml Kanamycin/ 0.25 mM IPTG resuspendiert und bei 22°C über Nacht kultiviert. Aus dem Überstand wurden die Phagen 2x mit PEG ausgefällt und durch Zentrifugation geerntet (Ausubel (1998) Current protocols in molecular biology. John Wiley Sons, Inc., New York, USA). Die Phagen wurden in PBS/ 20% Glyzerin resuspendiert und bei -80°C gelagert.

Die Phagemid-Amplifikation zwischen den einzelnen Selektionsrunden erfolgte folgendermaßen: log-Phase *E. coli* TG1-Zellen wurden mit den selektionierten Phagen infiziert und auf LB-Agar-Platten mit 1% Glucose/ 34 µg/ml Chloramphenicol ausplattiert. Nach Inkubation über Nacht wurden die Bakterienkolonien abgekratzt, neu kultiviert und mit VCSM13 Helferphagen infiziert.

### Primäre Selektion von Antikörpern gegen den Farbstoff Fuji 6-4 (ZK203468)

Die gereinigten und aufkonzentrierten Phagemide der HuCAL GOLD Antikörper-Bibliothek wurden in ein Standard-Selektionsverfahren eingesetzt. Als Antigene wurden BSA- bzw. Transferrin-gekoppeltes ZK203468 alternierend verwendet. Die Antigene wurden in PBS aufgenommen und in Konzentrationen von 50 µg/ml auf Maxisorp^{™} Mikrotiterplatten F96 (Nunc) aufgebracht. Die Maxisorp-Platten wurden über Nacht bei 4°C inkubiert ("coating"). Nach Abblocken der Maxisorp-Platten mit 5% Milchpulver in PBS wurden ca. 2E+13 HuCAL GOLD-Phagen in die Antigen-beladenen, abgeblockten Wells gegeben und dort über Nacht bzw. zwei Stunden bei Raumtemperatur inkubiert. Nach mehreren Waschschritten, die mit fortschreitenden Selektionsrunden stringenter wurden, wurden gebundene Phagen mit 20 mM DTT bzw. 100 µM unkonjugiertem ZK203468 eluiert. Insgesamt wurden drei aufeinanderfolgende Selektionsrunden durchgeführt, wobei die Phagenamplifikation zwischen den Selektionsrunden erfolgte, wie oben beschrieben.

### Sub-Klonierung selektionierter Fab-Fragmente zur Expression

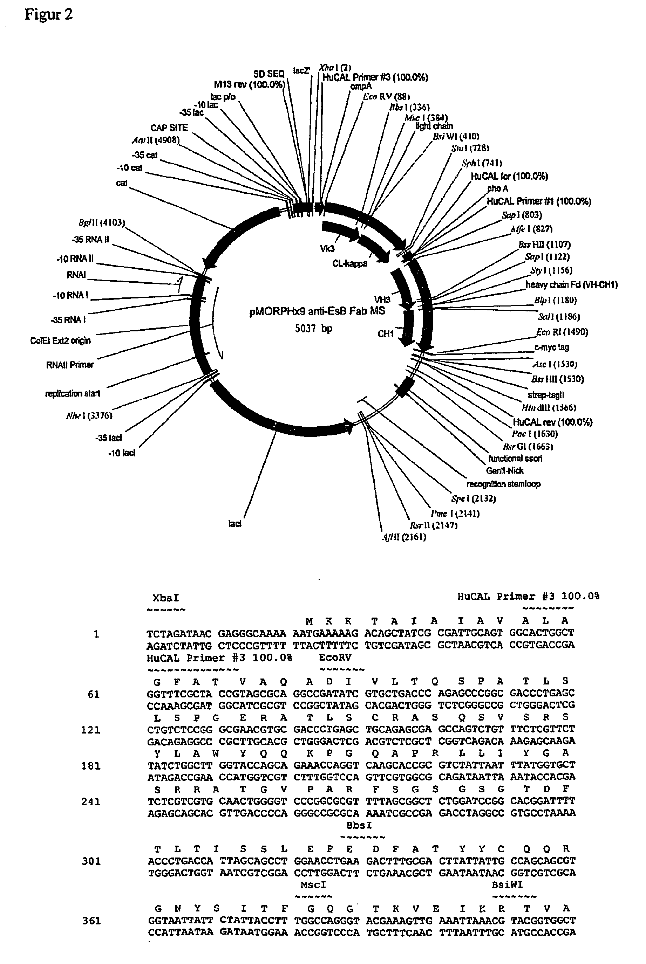

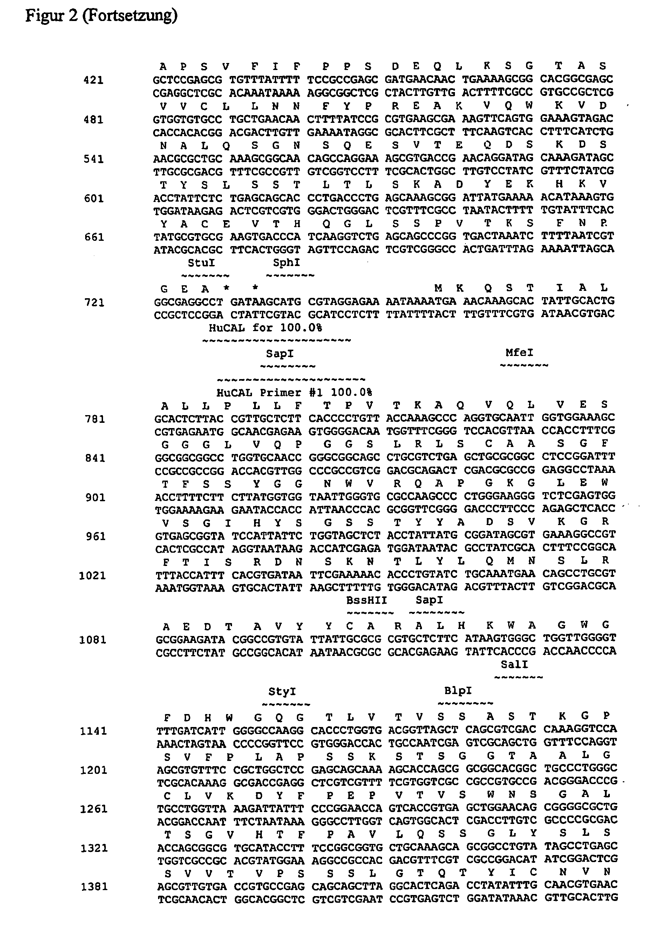

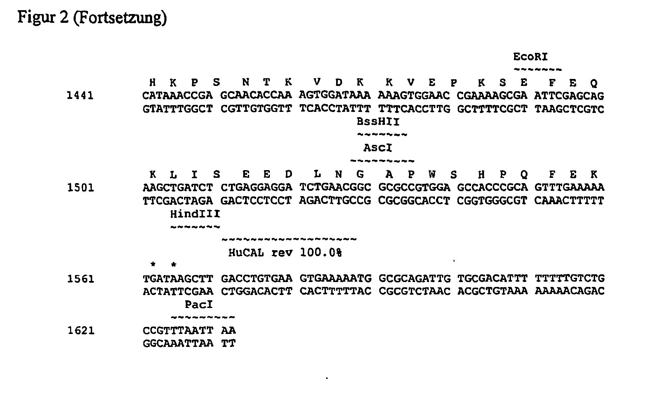

Nach der drei Runden umfassenden Antikörper-Selektion wurden die Fab-codierenden Inserts der isolierten HuCAL-Klone in den Expressionsvektor pMORPHX9_MS (s. Figur 2) sub-kloniert, um die anschließende Expression der Fab-Fragmente zu erleichtern. Dazu wurde die gereinigte Plasmid-DNA der selektionierten HuCAL Fab-Klone mit den Restriktionsenzymen XbaI und EcoRI verdaut. Die Fab-codierenden Inserts wurde aufgereinigt und in den entsprechend verdauten Vektor pMORPHX9 _MS ligiert. Dieser Klonierungsschritt führt zu dem Fab-exprimierenden Vektor pMORPHX9_Fab_MS. Fab-Fragmente, die von diesem Vektor exprimiert werden, tragen zwei C-terminale Tags (Myc-Tag und Strep-Tag II) zur Aufreinigung und Detektion.

### Screening und Charakterisierung von ZK203468-bindenden Fab-Fragmenten

Mehrere tausend Klone wurden nach der Selektion und Sub-Klonierung vereinzelt und mittels ELISA im 384-well-Format auf spezifische Erkennung der im Panning verwendeten Antigene ZK203468-BSA und -Transferrin getestet. Hierbei identifizierte Klone wurden in einem Inhibitions-ELISA auf effiziente Bindung des unkonjugierten Farbstoffs untersucht.

Dies führte zu den parenteralen Fab-Fragmenten MOR02628 (Proteinsequenzen SEQ-ID NO: 1 (VH-CH) und SEQ-ID NO: 2 (VL-CL); DNA-Sequenzen SEQ-ID NO: 3 (VH-CH) und SEQ-ID NO: 4 (VL-CL)), MOR02965 (Proteinsequenzen SEQ-ID NO: 5 (VH-CH) und SEQ-ID NO: 6 (VL-CL); DNA-Sequenzen SEQ-ID NO: 7 (VH-CH) und SEQ-ID NO: 8 (VL-CL)), MOR02977 (Proteinsequenzen SEQ-ID NO: 9 (VH-CH) und SEQ-ID NO: 10 (VL-CL); DNA-Sequenzen SEQ-ID NO: 11 (VH-CH) und SEQ-ID NO: 12 (VL-CL)) und MOR02969 (Proteinsequenzen SEQ-ID NO: 13 (VH-CH) und SEQ-ID NO: 14 (VL-CL); DNA-Sequenzen SEQ-ID NO: 15 (VH-CH) und SEQ-ID NO: 16 (VL-CL)), die den unkonjugierten Farbstoff ZK203468 effizient binden.

### Beispiel 2: Optimierung der parentalen Antikörper-Fragmenten durch Austausch der LCDR3-Region

### Klonierung der LCDR3-Bibliotheken

Die Plasmid-DNA der vier parenteralen Klone MOR02628, MOR02965, MOR02969 und MOR02977 wurde mit den Restriktionsenzymen EcoRI und XbaI verdaut und das entstandene, vollständige Fab-Insert vom Expressionsvektor pMORPHX9_MS in den entsprechend geschnittenen Display-Vektor pMORPH23 sub-kloniert. Dieser Schritt ist nötig, um das GenIII bereitzustellen, das der Präsentaion des Fab-Fragments auf der Phagenoberfläche dient. In einem weiteren Schritt wurden die vier parenteralen Klone (nun in pMORPH23) mit BpiI und SphI verdaut. Hierbei wurden die LCDR3-Region und der konstante Clambda-Bereich aus dem Vektor-Rückgrat entfernt. Das entsprechende Vektor-DNA-Fragment wurde isoliert und aufgereinigt. Parallel dazu wurde aus der HuCAL-Fab 2 Bibliothek das komplementäre BpiI/SphI-Fragment isoliert, welches eine diversifizierte LCDR3-Region (mit einer Variabilität von etwa 3E+8) plus konstanten Clambda-Bereich enthält (= Insert-DNA). Mehrere µg Vektor-DNA und kompatible Insert-DNA wurden im molaren Verhältnis von 1:2 mit T4-DNA-Ligase ligiert und nach einem Aufreinigungsschritt in elektrokompetente TOP10F'-Zellen transformiert. Dabei wurden pro parenteralem Antikörper Bibliotheksgrößen von 5E+8 bis etwa 1E+9 Klonen erzielt.

Die Bibliotheken, denen die Klone MOR02628, MOR02969 und MOR02977 zugrunde lagen, wurden vereinigt ("Pool"). Die MOR02965-Bibliothek wurde separat behandelt ("Lead"). Wie bereits beschrieben, wurden mittels dieser TOP10F'-Maturierungs-Bibliotheken die entsprechenden Phagemide durch Infektion mit VCSM13 Helferphagen produziert.

### Antikörper-Selektionen auf Maxisorp^{™} Mikrotiterplatten

Die gereinigten und aufkonzentrierten Phagemide von "lead"- und "pool"-Bibliotheken wurden in ein Maturierungs-Selektionsverfahren unter stringenten Bedingungen eingesetzt (lange Waschperioden, Verdrängung durch gereinigte, parentale Fab-Proteine). Als Antigene wurden BSA- bzw. Transferrin-gekoppeltes ZK203468 alternierend verwendet. Diese Antigene wurden in PBS aufgenommen und in geringen Konzentrationen von 100-250 ng/ml auf Maxisorp^{™} Mikrotiterplatten F96 (Nunc) aufgebracht. Die Maxisorp-Platten wurden über Nacht bei 4°C inkubiert ("coating"). Nach Abblocken der Maxisorp-Platten mit 5% Milchpulver in PBS wurden ca. 2E+11 HuCAL Phagen in die Antigen-beladenen, abgeblockten Wells gegeben und dort über Nacht bzw. zwei Stunden bei Raumtemperatur inkubiert. Um die Stringenz zu erhöhen, wurden während dieser Inkubation zusätzlich 100 nM bzw. 500 nM gereinigte Fab-Fragmente der parentalen Klone zugegeben. Nach mehreren ausgedehnten Waschschritten wurden gebundene Phagen mit 20 mM DTT eluiert. Insgesamt wurden zwei aufeinanderfolgende Selektionsrunden durchgeführt, wobei die Phagenamplifikation zwischen den Selektionsrunden erfolgte wie oben beschrieben.

### Antikörper-Selektionen auf Neutavidin-Strips

Die gereinigten und aufkonzentrierten Phagemide von "lead"- und "pool"-Bibliotheken wurden weiterhin in ein zweites Maturierungs-Selektionsverfahren unter stringenten Bedingungen (lange Waschperioden, Verdrängung durch aufgereinigte, parentale Fab-Proteine bzw. freien Farbstoff) eingesetzt. Als Antigene wurde Biotin-conjugiertes ZK203468 (alternierend mit Alkyl- bzw. Ether-Linker) verwendet. Diese Antigene wurden in PBS aufgenommen und in geringen Konzentrationen von 60 bzw. 12 ng/ml mit den ca. 2E+11 Phagen versetzt. Die Antigen-Phagen-Lösungen wurden über Nacht bzw. 2 Stunden bei Raumtemperatur inkubiert. Um die Stringenz zu erhöhen, wurden während dieser Inkubation zusätzlich 0.5 µg/ml gereinigte Fab-Fragmente der parentalen Klone bzw. 40 ng/ml ZK203468 zugegeben. Die Antigen-gebundene Phagen enthaltenden Lösungen wurden anschließend auf geblockte Neutravidin-Strips aufgebracht und 30 min inkubiert, um die Bindung an die Festphase über den Biotinrest des Antigens zu ermöglichen. Nach mehreren Waschschritten wurden gebundene Phagen mit 20 mM DTT eluiert. Insgesamt wurden zwei aufeinanderfolgende Selektionsrunden durchgeführt, wobei die Phagenamplifikation zwischen den Selektionsrunden erfolgte wie oben beschrieben.

### Sub-Klonierung selektionierter Fab-Fragmente zur Expression

Nach der Selektion ("Maturierung") wurden die Fab-codierenden Inserts der isolierten HuCAL-Klone in den Expressionsvektor pMORPHX9_MS sub-kloniert, um die anschließende Expression zu erleichtern. Dazu wurde die gereinigte Plasmid-DNA der selektionierten HuCAL Fab-Klone mit den Restriktionsenzymen XbaI und EcoRI verdaut. Das Fab-codierende Insert wurde aufgereinigt und in den entsprechend verdauten Vektor pMORPHX9_MS ligiert. Dieser Klonierungsschritt führt zu dem Fab-exprimierenden Vektor pMORFHX9_Fab_MS. Fab-Fragmente, die von diesem Vektor exprimiert werden, tragen zwei C-terminale Tags (Myc-Tag und Strep-Tag II) zur Aufreinigung und Detektion.

### Identifikation optimierter Antikörper-Fragmente

Um Antikörper mit verbesserten Affinitäten zum Farbstoff Fuji 6-4 zu identifizieren, wurden die Klone aus den Selektionen vereinzelt und in ELISAs im 384-well-Format gescreent. Dazu wurde auf die ELISA-Mikrotiterplatten ZK203468-BSA aufgebracht. Die zu untersuchenden Fab-Fragmente wurden als nicht-aufgereinigte Bakterienlysate zugegeben. Um zusätzlich zur Bindung an das Antigenkonjugat die Bindung an den freien Farbstoff zu untersuchen, wurden ausserdem identische Screeningplatten mit Bakterienlysat und zusätzlich freiem Farbstoff in zwei unterschiedlichen Konzentrationen versetzt. Die hierbei entstehende Inhibition der Fab-Bindung an die Festphase durch den unkonjugierten Farbstoff zeigte Antikörper an, die nicht das Farbstoff-Konjugat sondern nur den freien Farbstoff spezifisch erkennen. Hierbei isolierte Klone wurden in Solution-Inhibition-Tests im ELISA-Format und dem Luminex-Gerät genau charakterisiert und deren Affinitäten zu Fuji 6-4 bestimmt.

Die folgenden Klone zeigten verbesserte Affinitäten im Vergleich zu den parenteralen Antikörpern:

| Name | Parenteraler Fab | LCDR3-Sequenz |
|---|---|---|
| **MOR02969** - | | SSYTYRVGGM |
| **MOR03291** | MOR02969 | ASYDYKSKNI |
| **MOR02965** - | | SS**WD**SSFS**W** |
| **MOR03263** | MOR02965 | SS**WD**VSLE**W** |
| **MOR02977** - | | QS**W**TTRPLN**R** |
| **MOR03201** | MOR02977 | SS**W**TSYFHI**R** |
| **MOR03267** | MOR02977 | QA**WD**SNFKN**R** |
| **MOR03292** | MOR02977 | QS**W**APLFKM**R** |
| **MOR03295** | MOR02977 | QS**WD**SALSN**R** |

Zusammenfassend konnte die Affinität des parenteralen MOR02977 im Vergleich zu MOR03267 um den Faktor 140 verbessert werden. Alle weiteren identifizierten Klone zeigten Verbesserungen von Faktor 2-70 im Vergleich zum jeweiligen parenteralen Fab.

### Beispiel 3: Photophysikalische Charakterisierung der Farbstoff-Antikörper-Komplexe und Bestimmung der spektralen Verschiebungen / Fluoreszenzquantenausbeuten

Es wurden Farbstoff-Antikörper-Komplexe basierend auf Antikörpern mit Bindung an den Indotricarbocyaninfarbstoff Trinatrium-3,3-dimethyl-2-{4-methyl-7-[3,3-dimethyl-5-sulfonato-1-(2-sulfonatoethyl)-3H-indolium-2-yl]hepta-2,4,6-trien-1-yliden} -1-(2-sulfonatoethyl)-2,3-dihydro-1H-indol-5-sulfonat, inneres Salz untersucht (siehe Beispiele 1 und 2). Es wurden Lösungen der Konzentration von 1 µmol/L des o. g. Farbstoffes und 2,4 µmol/L des jeweiligen Antikörpers in PBS hergestellt und 2 h bei Raumtemperatur inkubiert. Die Absorptionsmaxima wurden mit einem Spektralphotometer (Perkin-Elmer, Lambda2) bestimmt. Die Fluoreszenzmaxima und Fluoreszenzquantenausbeuten wurden mit einem SPEX Fluorolog (wellenlängenabhängige Empfindlichkeit von Lampe und Detektor kalibriert) relativ zu Indocyaningrün bestimmt (Q = 0,13 in DMSO, J Chem Eng Data 1977, 22, 379, Bioconjugate Chem 2001, 12, 44). Aus den Absorptions- und Fluoreszenzmaxima wurden die spektralen Verschiebungen relativ zu den Maxima einer Lösung des o. g. Farbstoffes ohne Antikörper in PBS (1 µmol/L) berechnet (Absorptionsmax. 754 nm, Fluoreszenzmax. 783 nm, Fluoreszenzquantausbeute 10 %).

| Name | Parenteraler Fab | LCDR3-Sequenz | SEQ-ID NO. VH Protein | SEQ-ID NO. VL Protein | SEQ-ID NO. VH DNA | SEQ-ID NO. VL DNA |
|---|---|---|---|---|---|---|
| | **MOR02628** | AAWDFRKRL N | 1 | 2 | 3 | 4 |
| | **MOR02965** | SS**WD**SSFS**W** | 5 | 6 | 7 | 8 |
| | **MOR02977** | QS**W**TTRPLN **R** | 9 | 10 | 11 | 12 |
| | **MOR02969** | SS**Y**TYRVGG M | 13 | 14 | 15 | 16 |
| **MOR02965** - | | SS**WD**SSFS**W** | 5 | 6 | 7 | 8 |
| **MOR03263** | MOR02965 | SS**WD**VSLE**W** | 5 | 17 | 7 | 18 |
| **MOR03325** | MOR02965 | AS**WD**KSLQ **W** | 5 | 19 | 7 | 20 |
| **MOR03285** | MOR02965 | QA**W**TGSYAT | 5 | 37 | 7 | 38 |
| **MOR02977** - | | QS**W**TTRPLN **R** | 9 | 10 | 11 | 12 |
| **MOR03201** | MOR02977 | SS**W**TSYFHI**R** | 9 | 21 | 11 | 22 |
| **MOR03267** | MOR02977 | Q**AWD**SNFKN **R** | 9 | 23 | 11 | 24 |
| **MOR03268** | MOR02977 | RS**WD**SNLSY S | 9 | 25 | 11 | 26 |
| **MOR03292** | MOR02977 | QS**W**APLFKM **R** | 9 | 27 | 11 | 28 |
| **MOR03294** | MOR02977 | QT**W**TSSFSS**R** | 9 | 29 | 11 | 30 |
| **MOR03295** | MOR02977 | QS**WD**SALSN **R** | 9 | 31 | 11 | 32 |
| **MOR03309** | MOR02977 | QT**WD**HGFTH **R** | 9 | 33 | 11 | 34 |
| **MOR03293** | MOR02977 | SS**W**TTIYRN **R** | 9 | 39 | 11 | 40 |
| **MOR02969** - | | SS**Y**TYRVGG M | 13 | 14 | 15 | 16 |
| **MOR03291** | MOR02969 | AS**YD**YKSKN I | 13 | 35 | 15 | 36 |

Die Ergebnisse sind in folgender Tabelle zusammengefaßt:

| Antikörper | Parenteraler Antikörper | Absorptions-maximum (nm) | Fluoreszenz-maximum (nm) | Absorptions-shift (nm) | Fluoreszenz-shift (nm) | Fluoreszenz-quantenausbeute (%) |
|---|---|---|---|---|---|---|
| freier Farbstoff - | | 754 | 783 | - | - | 10,0 |
| MOR02965 | - | 799 | 815 | 45 | 32 | 13,0 |
| MOR03263 | MOR02965 | 798 | 810 | 44 | 27 | 10,0 |
| MOR03325 | MOR02965 | 803 | 819 | 49 | 36 | 18,0 |
| MOR02977 | - | 773 | 788 | 19 | 5 | 24,5 |
| MOR03201 | MOR02977 | 786 | 804 | 32 | 21 | 21,0 |
| MOR03267 | MOR02977 | 783 | 802 | 29 | 19 | 38,5 |
| MOR03268 | MOR02977 | 786 | 805 | 32 | 22 | 30,0 |
| MOR03292 | MOR02977 | 781 | 800 | 27 | 17 | 25,0 |
| MOR03294 | MOR02977 | 783 | 803 | 29 | 20 | 22,0 |
| MOR03295 | MOR02977 | 784 | 803 | 30 | 20 | 23,0 |
| MOR03309 | MOR02977 | 784 | 803 | 30 | 20 | 21,5 |

### Beispiel 4: Konstruktion von Expressionsvektoren für die Expression von HuCAL Immunoglobulinen

Klonierung der schweren Kette: Die "multiple cloning site" des Vektors pCDNA3.1+ (Invitrogen) wird entfernt (NheI/Apal), und ein Platzhalter, der mit den Restriktionsschnittstellen aus dem HuCAL-Design kompatibel ist, wird eingesetzt für die Ligation der Leader-Sequenz (NheI/EcoRI), der VH-Domäne aus dem Fab-Fragment (MunI/), und die konstanten Immunoglobulinregionen (BlpI/ApaI). Die Leadersequenz (EMBL 83133) wird mit einer Kozaksequenz ausgestattet (Kozak, 1987). Die konstanten Regionen von humanem IgG (PIR J00228), IgG4 (EMBL K01316), und Serum-IgAl (EMBL J00220) werden in überlappende Oligonucleotide von einer Länge von etwa 70 Basen aufgeteilt. "Silent Mutations" werden eingeführt, um Restriktionsschnittstellen zu entfernen, die nicht mit dem HuCAL-Design kompatibel sind. Die Oligonukleotide werden durch "overlap extension-PCR" verknüpft.

Während des Subklonierens von den Fab-Fragmenten in ein IgG-Molekül wird die schwere Kette des Fab-Fragments über MfeI/BlpI ausgeschnitten und in den Vektor, der mit Eco-RI/BlpI geöffnet wird, ligiert. EcoRI (g/aattc) und MfeI (c/aattg) besitzten beide kompatible kohäsive Enden (aatt), und die Sequenz der ursprünglichen MfeI-Schnittstelle in den Fab-Fragmenten ändert sich von: c/aattg nach g/aattg nach der Ligation in den IgG-Expressionsvektor, wodurch zum Einen sowohl die MfeI- als auch die EcoRI-Schnittstelle zerstört werden, und zum Anderen eine Aminosäurenaustausch von Q (Kodon: caa) nach E (Kodon: gaa) statt findet.

Klonierung der leichten Kette. Die "multiple cloning site" von pCDNA3.1/Zeo+ (Invitrogen) wird duch zwei unterschiedliche Platzhalter ersetzt. Der k-Platzhalter enthält Restriktionsschnittstellen für den Einbau einer k-Leadersequenz (NheI/EcoRV), der HuCAL Fab Vk-Domäne (EcoRV/BsiWI), und der konstanten Region der k-Kette (BsiWI/ApaI). Die entsprechenden Schnittstellen im 1-Platzhalter sind NheI/EcoRV (1-Leader), EcoRV/HpaI (V1-Domäne), and HpaI/ApaI (konstante Region 1-Kette). Der k-Leader (EMBL Z00022) sowie der 1-Leader (EMBL J00241) sind beide mit Kozak-Sequenzen versehen. Die konstanten Regionen von humanen k- (EMBL L00241) und 1-Ketten (EMBL M18645) werden beide durch "overlap extension-PCR" assembliert, so wie oben beschrieben.

Generation von IgG-exprimierenden CHO-Zellen. CHO-K1 Zellen werden mit einer äquimolaren Mischung von Expressionsvektoren für die schwere und leichte IgG-Ketten cotransfiziert. Zweifach resistente Transfektantent werden mit 600 mg/ml G418 und 300 mg/ml Zeocin (Invitrogen) gefolgt von limitierender Verdünnung selektioniert. Der Überstand von Einzelklonen wird auf IgG-Expression durch "capture-ELISA" überprüft. Positive Klone werden in RPMI-1640 Medium, das mit 10% "ultra-low IgG-FCS" (Life Technologies) versehen ist, angezogen. Nach Einstellung des pH-Werts des Überstands auf 8.0 und Sterilfiltration, wird die Lösung einer Standard-ProteinA-Säulenchromatographie unterzogen (Poros 20 A, PE Biosystems).

## Patentansprüche

1. Emitter-bindendes Peptid, **dadurch gekennzeichnet, daß** dieses bei einer Wechselwirkung seiner Antigenbindungstasche mit dem Emitter eine Veränderung der spektralen Emissionseigenschaften des Emitters bewirkt,
wobei der Emitter einen Farbstoff umfaßt, der mindestens ein Absorptionsmaximum und/oder Fluoreszenzmaximum innerhalb des Spektralbereiches von 700 bis 1000 nm, bevorzugt mindestens ein Absorptionsmaximum und Fluoreszenzmaximum innerhalb des Spektralbereiches von 750 bis 900 nm aufweist,
wobei der Farbstoff Polymethinfarbstoff ist, und
wobei das Emitterbindende Peptid ein Antikörper oder Antikörperfragment ist.

2. Emitter-bindendes Peptid nach Anspruch 1, wobei Antikörper oder Antikörperfragmente Fab-Fragmenten, scFv-Fragmenten, scTCR-Ketten, single-chain-Antikörpern oder Gemische davon umfassen.

3. Emitter-bindendes Peptid nach Anspruch 2, umfassend eines der VH/VL-Paare, das in einem der folgenden Sequenzpaare enthalten ist: SEQ-ID NOs: 1+2; SEQ-ID NOs: 5+6; SEQ-ID NOs: 9+10; SEQ-ID NOs: 13+14; SEQ-ID NOs: 5+17; SEQ-ID NOs: 5+19; SEQ-ID NOs: 5+37; SEQ-ID NOs: 9+21; SEQ-ID NOs: 9+23; SEQ-ID NOs: 9+25; SEQ-ID NOs: 9+27; SEQ-ID NOs: 9+29; SEQ-ID NOs: 9+31; SEQ-ID NOs: 9+33; SEQ-ID NOs: 9+39; und SEQ-ID NOs: 13+35.

4. Emitter-bindendes Peptid nach einem der Ansprüche 1 bis 3, dessen Bindungsaffinität zu dem Emitter kleiner als 50 nM und bevorzugt kleiner als 10 nM ist.

5. Emitter-bindendes Peptid nach einem der Ansprüche 1 bis 4, wobei die Veränderung der Emissionseigenschaften des Emitters ausgewählt ist aus einer Veränderung der Polarisationsebene der Fluoreszenzintensität, Phosphoreszenz, insbesondere Phosphoreszenzintensität, Lebensdauer der Fluoreszenz und einer bathochromen Verschiebung des Absorptionsmaximums und/oder Fluoreszenzmaximums.

6. Emitter-bindendes Peptid nach einem der Ansprüche 1 bis 5, wobei die Verschiebung des Absorptions- und/oder Fluoreszenzmaximums zu höheren Wellenlängen nach Wechselwirkung mit dem Mittel zur Erkennung des Emitters um einen Wert größer 15 nm, bevorzugt größer 25 nm, und am meisten bevorzugt um ungefähr 30 nm erfolgt.

7. Emitter-bindendes Peptid nach einem der Ansprüche 1 bis 6, wobei Polymethinfarbstoff ausgewählt ist aus der Gruppe von Dicarbocyanin-, Tricarbocyanin-, Indotricarbocyanin-, Merocyanin-, Styryl-, Squarilium- und Oxonolfarbstoffen und Rhodaminfarbstoffen, Phenoxazin- oder Phenothiazinfarbstoffen.

8. Emitter-bindendes Peptid nach einem der Ansprüche 1 bis 7, wobei der Farbstoff ein Cyaninfarbstoff der allgemeinen Formel (I) 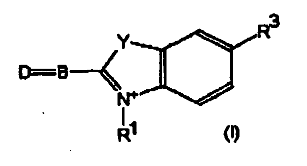 umfaßt, in dem D für einen Rest (II) oder (III) 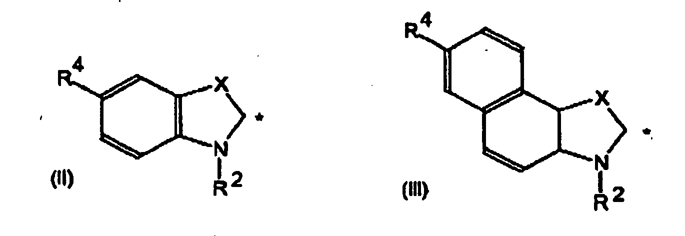 steht, wobei die mit dem Stern markierte Position die Verknüpfungsstelle mit dem Rest B bedeutet und B für die Gruppe (IV), (V), (VI), (VII) oder (VIII) 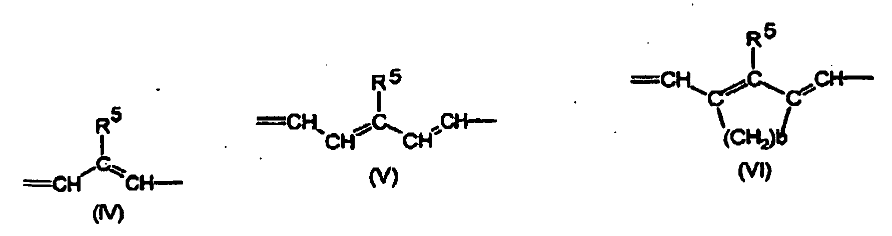 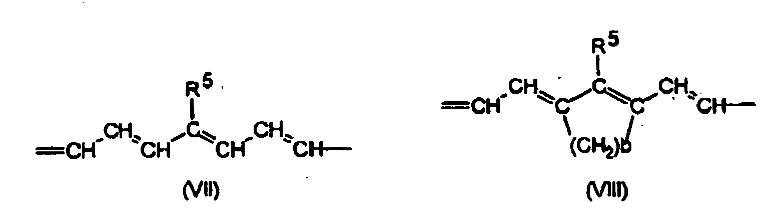 stehen kann in denen R¹ und R² unabhängig voneinander eine C₁-C₄-Sulfoalkylkette, eine gesättigte oder ungesättigte, verzweigte oder geradekettige C₁-C₅₀-Alkylkette darstellt, die gegebenenfalls mit von 0 bis 15 Sauerstoffatomen und/oder von 0 bis 3 Carbonylgruppen unterbrochen ist und/oder mit 0 bis 5 Hydroxygruppen substituiert sein kann, bedeutet,
R¹ und R⁴ unabhängig voneinander für die Gruppe -COOE¹, -CONE¹E², -NHCOE¹, - NHCONHE¹, -NE¹E², -OE¹, -OSO₃E¹, -SO₃E¹, -SO₂NHE¹ oder -E¹ steht, wobei E¹ und E² unabhängig voneinander ein Wasserstoffatom, eine C₁-C₄-Sulfolkylkette, eine gesättigte oder ungesättigte, verzweigte oder geradekettige C₁-C₃₀-Alkylkette darstellt, die gegebenenfalls von 0 bis 15 Sauerstoffatomen und/oder von 0 bis 3 Carbonylgruppen unterbrochen ist und/oder mit 0 bis 5 Hydroxygruppen substituiert ist,
R⁵ für ein Wasserstoffatom oder ein Fluor- Chlor, Brom- oder Jodatom, Me, Et, Prop steht,
b die Zahl 2 oder 3 bedeutet, und
X O, S, =C(CH₃)₂ oder-(CH=CH)- bedeutet, sowie Salz und Solvate dieser Verbindungen.

9. Polynukleotid, insbesondere DNA, RNA oder PNA, umfassend eine Sequenz, die für ein Emitter-bindendes Peptid nach einem der Ansprüche 1 bis 8 oder funktionellen Varianten davon kodiert.

10. DNA- oder RNA-Vektormolekül, das mindestens ein oder mehrere Polynukleotid(e) nach Anspruch 9 enthält und das in Zellen exprimierbar ist.

11. Wirtszelle, die ein Polynukleotid gemäß Anspruch 9 oder ein Vektormolekül gemäß Anspruch 10 enthält.

12. Antikörper, insbesondere polyklonaler oder monoklonaler Antikörper, humaner oder humanisierter Antikörper, synthetischer oder rekombinanter Antikörper, umfassend mindestens ein Emitter-bindendes Peptid nach einem der Ansprüche 1 bis 8.

13. Verfahren zur Herstellung eines Emitter-bindenden Peptids nach einem der Ansprüche 1 bis 8, umfassend die Immunisierung eines geeigneten Organismus mit einem Emitter, umfassend einen Polymethinfarbstoff, der ausgewählt ist aus der Gruppe Dicarbocyanin-, Tricarbocyanin-, Indotricarbocyanin-, Merocyanin-, Styryl-, Squarilium- und Oxonolfabstoffen und Rhodanünfarbstoffen, Phenoxazin- oder Phenothiazinfarbstof fen.

14. Verfahren zur Herstellung eines Emitter-bindenden Peptids nach einem der Ansprüche 1 bis 8, umfassend die rekombinante und/oder synthetische Herstellung des Peptids.

15. Verwendung eines Emitter-bindenden Peptids nach einem der Ansprüche 1 bis 8, einer Nukleinsäure nach Anspruch 9 oder 10, einer Wirtszelle nach Anspruch 11, oder eines Antikörpers nach Anspruch 12 als Diagnostikum für die *in vitro* Diagnostik.

16. Diagnostischer Kit für die *in vitro* Diagnose, umfassend mindestens ein Mittel ausgewählt aus einem Emitter-bindenden Peptids nach einem der Ansprüche 1 bis 8, einer Nukleinsäure nach Anspruch 9 oder 10, einer Wirtszelle nach Anspruch 11, oder einem Antikörper nach Anspruch 12, gegebenenfalls zusammen mit anderen Hilfsstoffen und/oder Anweisungen, in gemeinsamen oder in getrennten Behältern.

17. Verfahren zur quantitativen in vilro-Bestimmung einer in einer Probe enthaltenen Substand, umfassend den Schritt von,
a) in Kontakt bringen eines Emitter-bindenden Peptids nach einem der Ansprüche 1 bis 8 mit einem Emitter, wobei die Wechselwirkung des Emitters des Konjugats mit dem Emitter-bindenden Peptid eine Veränderung der spektralen Emissionseigenschatten des Emitters bewirkt, und
b) Messen der Veränderung der spektralen Emissionseigenschaften des Emitters.

18. Verfahren zur direkten quantitativen in vitro-Bestimmung einer in einer Probe enthaltenen Substanz oder eines in der Probe vorhandenen Antigen-erkennenden Mittels nach Anspruch 17, umfassend weiterhin den Schritt von,
d) Quantifizieren der in der Probe enthaltenen Substanz mittels der gemessenen Veränderung der Emissionseigenschaften des Emitters.

19. Verfahren nach Anspruch 17 oder 18, wobei die Veränderung der spektralen Emissionseigenschaften des Teils des Emitters ausgewählt ist aus einer Veränderung der Polarisationsebene, Phosphoreszenz, insbesondere Phosphoreszenzintensität, Lebensdauer der Fluoreszenz und einer bathochromen Verschiebung des Absorptionsmaximums und/oder Fluoreszenzmaximums.

20. Verfahren nach einem der Ansprüche 17 bis 19, wobei als das Emitter-bindende Peptid, Antikörperfragmente, wie zum Beispiel Fab-Fragmente, scFv-Fragmente, scTCR-Ketten, single-chain-Antikörper und Gemische davon mit der Probe in Kontakt gebracht werden.

21. Verfahren nach einem der Ansprüche 17 bis 20, wobei das Emitter-bindende Peptid eine Sequenz gemäß Anspruch 3 umfaßt.

22. Verfahren nach einem der Ansprüche 17 bis 21, wobei das Emitter-bindende Peptid eine Bindungsafffmität zu dem Emitter von weniger als 50 nM und bevorzugt weniger als 10 nM aufweist.

## Claims

1. Emitter-binding peptide, **characterized in that** the latter produces a change in the spectral emission properties of the emitter in the case of an interaction of its antigen binding pocket with the emitter, wherein the emitter comprises a dye that has at least an absorption maximum and/or fluorescence maximum within the spectral range of 700 to 1000 nm, preferably at least an absorption maximum and fluorescence maximum within the spectral range of 750 to 900 nm, wherein the dye is polymethine dye, and wherein the emitter-binding peptide is an antibody or antibody fragment.

2. Emitter-binding peptide according to Claim 1, wherein antibodies or antibody fragments comprise Fab fragments, scFv fragments, scTCR chains, single-chain antibodies or mixtures thereof.

3. Emitter-binding peptide according to Claim 2, comprising one of the VH/VL pairs, which is contained in one of the following sequence pairs: SEQ-ID Nos.: 1+2; SEQ-ID Nos.: 5+6; SEQ-ID Nos.: 9+10; SEQ-ID Nos.: 13+14; SEQ-ID Nos.: 5+17; SEQ-ID Nos.: 5+19; SEQ-ID Nos.: 5+37; SEQ-ID Nos.: 9+21; SEQ-ID Nos.: 9+23; SEQ-ID Nos.: 9+25; SEQ-ID Nos.: 9+27; SEQ-ID Nos.: 9+29; SEQ-ID Nos.: 9+31; SEQ-ID Nos.: 9+33; SEQ-ID Nos.: 9+39; and SEQ-ID Nos.: 13+35.

4. Emitter-binding peptide according to one of Claims 1 to 3, whose binding affinity for the emitter is less than 50 nM and preferably less than 10 nM.

5. Emitter-binding peptide according to one of Claims 1 to 4, wherein the change in the emission properties of the emitter is selected from a change in the polarization plane of the fluorescence intensity, phosphorescence, especially phosphorescence intensity, service life of the fluorescence and a bathochromic shift of the absorption maximum and/or fluorescence maximum.

6. Emitter-binding peptide according to one of Claims 1 to 5, wherein the shift of the absorption and/or fluorescence maximum to higher wavelengths after interaction with the agent to detect the emitter is carried out by a value of greater than 15 nm, preferably greater than 25 nm, and most preferably by approximately 30 nm.

7. Emitter-binding peptide according to one of Claims 1 to 6, wherein polymethine dye is selected from the group of dicarbocyanine, tricarbocyanine, indotricarbocyanine, merocyanine, styryl, squarilium and oxonol dyes and rhodamine dyes, phenoxazine or phenothiazine dyes.

8. Emitter-binding peptide according to one of Claims 1 to 7, wherein the dye comprises a cyanine dye of general formula (I) 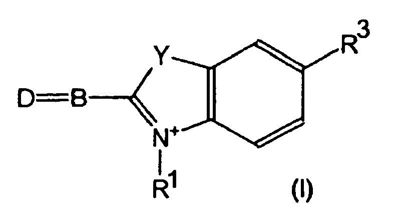 in which D stands for a radical (II) or (III) 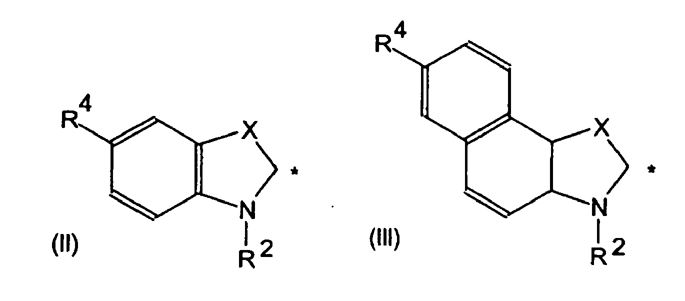 whereby the position that is labeled with the star means the point of linkage with radical B and B can stand for the group (IV), (V), (VI), (VII) or (VIII) 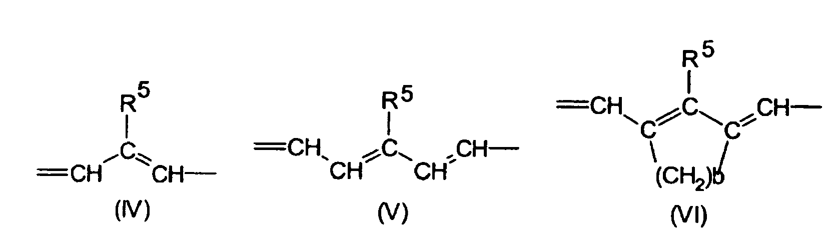 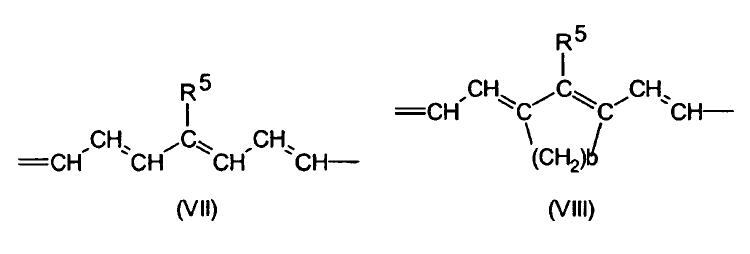
in which R¹ and R², independently of one another, represent a C₁-C₄-sulfoalkyl chain, a saturated or unsaturated, branched or straight-chain C₁-C₅₀-alkyl chain, which optionally is interrupted by 0 to 15 oxygen atoms and/or by 0 to 3 carbonyl groups and/or can be substituted with 0 to 5 hydroxy groups;
R³ and R⁴, independently of one another, stand for the group -COOE¹, -CONE¹E², -NHCOE¹, -NHCONHE¹, -NE¹E², -OE¹, -OSO₃E¹, -SO₃E¹, -SO₂NHE¹ or -E¹, whereby E¹ and E², independently of one another, represent a hydrogen atom, a C₁-C₄-sulfoalkyl chain, a saturated or unsaturated, branched or straight-chain C₁C₅₀-alkyl chain, which optionally is interrupted by 0 to 15 oxygen atoms and/or by 0 to 3 carbonyl groups and/or is substituted with 0 to 5 hydroxy groups, R⁵ stands for a hydrogen atom, or a fluorine, chlorine, bromine or iodine atom, Me, Et, or Prop,
b means the number 2 or 3, and
X is O, S, =C(CH₃)₂ or -(CH=CH)-, as well as salt and solvates of these compounds.

9. Polynucleotide, especially DNA, RNA or PNA, comprising a sequence that codes for an emitter-binding peptide according to one of Claims 1 to 8 or functional variants thereof.

10. DNA- or RNA-vector molecule, which contains at least one or more polynucleotide(s) according to Claim 9 and which can be expressed in cells.

11. Host cell that contains a polynucleotide according to Claim 9 or a vector molecule according to Claim 10.

12. Antibodies, especially polyclonal or monoclonal antibodies, human or humanized antibodies, synthetic or recombinant antibodies, comprising at least one emitter-binding peptide according to one of Claims 1 to 8.

13. Process for the production of an emitter-binding peptide according to one of Claims 1 to 8, comprising the immunization of a suitable organism with an emitter, comprising a polymethine dye, which is selected from the group of dicarbocyanine, tricarbocyanine, indotricarbocyanine, merocyanine, styryl, squarilium and oxonol dyes and rhodamine dyes, phenoxazine or phenothiazine dyes.

14. Process for the production of an emitter-binding peptide according to one of Claims 1 to 8, comprising the recombinant and/or synthetic production of the peptide.

15. Use of an emitter-binding peptide according to one of Claims 1 to 8, a nucleic acid according to Claim 9 or 10, a host cell according to Claim 11, or an antibody according to Claim 12 as a diagnostic agent for *in vitro* diagnosis.

16. Diagnostic kit for *in vitro* diagnosis, comprising at least one agent that is selected from an emitter-binding peptide according to one of Claims 1 to 8, a nucleic acid according to Claim 9 or 10, a host cell according to Claim 11, or an antibody according to Claim 12, optionally together with other adjuvants and/or instructions, in common or in separate containers.

17. Process for quantitative *in vitro* determination of a substance that is contained in a sample, comprising the steps of
a) bringing into contact an emitter-binding peptide according to one of Claims 1 to 8 with an emitter, whereby the interaction of the emitter of the conjugate with the emitter-binding peptide produces a change in the spectral emission properties of the emitter, and
b) measuring the change in the spectral emission properties of the emitter.

18. Process for direct quantitative *in vitro* determination of a substance that is contained in a sample or an antigen-detecting agent that is present in the sample according to Claim 17, in addition comprising the step of,
d) quantification of the substance that is contained in the sample by means of the measured change in the emission properties of the emitter.

19. Process according to Claim 17 or 18, wherein the change in the spectral emission properties of the part of the emitter is selected from a change of the polarization plane, phosphorescence, especially phosphorescence intensity, service life of the fluorescence and a bathochromic shift of the absorption maximum and/or fluorescence maximum.

20. Process according to one of Claims 17 to 19, wherein as the emitter-binding peptide, antibody fragments such as for example Fab fragments, scFv fragments, scTCR chains, single-chain-antibodies and mixtures thereof are brought into contact with the sample.

21. Process according to one of Claims 17 to 20, whereby the emitter-binding peptide comprises a sequence according to Claim 3.

22. Process according to one of Claims 17 to 21, wherein the emitter-binding peptide has a binding affinity for the emitter of less than 50 nM and preferably less than 10 nM.

## Revendications

1. Peptide liant un émetteur, **caractérisé en ce que** celui-ci provoque une modification des propriétés spectrales d'émission lors d'une interaction de sa poche de liaison à l'antigène avec l'émetteur,
- où l'émetteur comprend un colorant qui présente au moins un maximum d'absorption et/ou de fluorescence dans la plage spectrale de 700 à 1000 nm, de préférence au moins un maximum d'absorption et au moins un maximum de fluorescence dans la plage spectrale de 750 à 900 nm,
- où le colorant est un colorant de type polyméthine, et
- où le peptide liant l'émetteur est un anticorps ou un fragment d'anticorps.

2. Peptide liant un émetteur selon la revendication 1, où les anticorps ou les fragments d'anticorps comprennent des fragments Fab, des fragments scFv, des chaînes scTCR, des anticorps à chaîne simple ou leurs mélanges.

3. Peptide liant un émetteur selon la revendication 2, comprenant une des paires VH/VL, qui est contenue dans l'une quelconque des paires de séquences suivantes : SEQ-ID NO : 1 + 2 ; SEQ-ID NO : 5 + 6 ; SEQ-ID NO : 9 + 10 ; SEQ-ID NO : 13 + 14 ; SEQ-ID NO : 5 + 17 ; SEQ-ID NO : 5 + 19 ; SEQ-ID NO : 5 + 37 ; SEQ-ID NO : 9 + 21 ; SEQ-ID NO : 9 + 23 ; SEQ-ID NO : 9 + 25 ; SEQ-ID NO : 9 + 27 ; SEQ-ID NO : 9 + 29 ; SEQ-ID NO : 9 + 31 ; SEQ-ID NO : 9 + 33 ; SEQ-ID NO : 9 + 39 ; et SEQ-ID NO : 13 + 35.

4. Peptide liant un émetteur selon l'une quelconque des revendications 1 à 3, dont l'affinité de liaison avec l'émetteur est inférieure à 50 nM et de préférence inférieure à 10 nM.

5. Peptide liant un émetteur selon l'une quelconque des revendications 1 à 4, où la modification des propriétés d'émission de l'émetteur est choisie parmi une modification du plan de polarisation de l'intensité de fluorescence, de la phosphorescence, en particulier de l'intensité de phosphorescence, de la durée de vie de la fluorescence et un déplacement bathochrome du maximum d'absorption et/ou de fluorescence.

6. Peptide liant un émetteur selon l'une quelconque des revendications 1 à 5, où le déplacement du maximum d'absorption et/ou de fluorescence se produit vers les longueurs d'onde plus élevées après l'interaction avec l'agent pour la détection de l'émetteur d'une valeur supérieure à 15 nm, de préférence supérieure à 25 nm, et de manière particulièrement préférée d'environ 30 nm.

7. Peptide liant un émetteur selon l'une quelconque des revendications 1 à 6, où le colorant de type polyméthine est choisi dans le groupe formé par les colorants de type dicarbocyanine, tricarbocyanine, indotricarbocyanine, mérocyanine, styryle, squarilium et oxonol et les colorants de type rhodamine, phénoxazine ou phénothiazine.

8. Peptide liant un émetteur selon l'une quelconque des revendications 1 à 7, où le colorant comprend un colorant de type cyanine de formule générale (I) 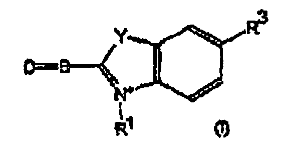 dans lequel D représente un radical (II) ou (III) 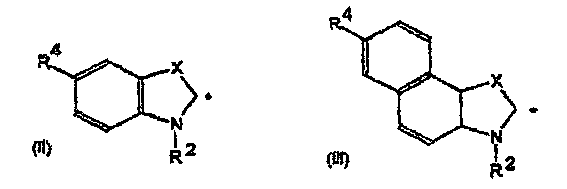 où la position marquée par une étoile signifie le site de liaison avec le radical B et B peut représenter le groupe (IV), (V), (VI), (VII) ou (VIII) 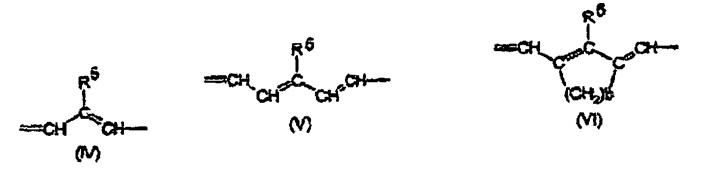 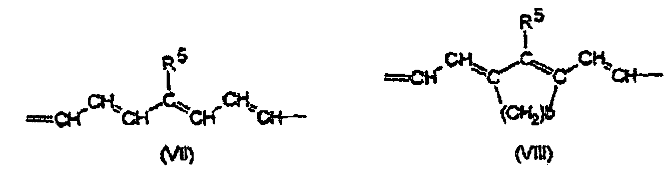
où R¹et R² représentent, indépendamment l'un de l'autre, une chaîne C₁-C₄-sulfoalkyle, une chaîne C₁-C₅₀-alkyle saturée ou insaturée, ramifiée ou linéaire, qui est le cas échéant interrompue par 0 à 15 atomes d'oxygène et/ou 0 à 3 groupes carbonyle et/ou qui peut être substituée par 0 à 5 groupes hydroxy,
R³ et R⁴ représentent indépendamment l'un de l'autre le groupe -COOE¹, -CONE¹E², -NHCOE¹, -NHCONHE¹, -NE¹E², -OE¹, -OSO₃E¹, SO₃E¹, -SO₂NHE¹ ou -E¹ où
E¹ et E² représentent, indépendamment l'un de l'autre, un atome d'hydrogène, une chaîne C₁-C₄-sulfoalkyle, une chaîne C₁-C₅₀-alkyle saturée ou insaturée, ramifiée ou linéaire, qui est le cas échéant interrompue par 0 à 15 atomes d'oxygène et/ou 0 à 3 groupes carbonyle et/ou substituée par 0 à 5 groupes hydroxy,
R⁵ représente un atome d'hydrogène ou un atome de fluor, de chlore, de brome ou d'iode, Me, Et, prop,
b vaut le nombre 2 ou 3, et
X signifie O, S, =C(CH₃)₂ ou -(CH=CH)-, ainsi qu'un sel et les solvates de ces composés.

9. Polynucléotide, en particulier ADN, ARN ou APN, comprenant une séquence qui code pour un peptide liant un émetteur selon l'une quelconque des revendications 1 à 8 ou des variantes fonctionnelles de celui-ci.

10. Molécule de vecteur d'ADN ou d'ARN qui contient au moins un ou plusieurs polynucléotide(s) selon la revendication 9 et qui peut être exprimée dans des cellules.

11. Cellule hôte qui contient un polynucléotide selon la revendication 9 ou une molécule de vecteur selon la revendication 10.

12. Anticorps, en particulier anticorps polyclonal ou monoclonal, anticorps humain ou humanisé, anticorps synthétique ou recombinant, comprenant au moins un peptide liant un émetteur selon l'une quelconque des revendications 1 à 8.

13. Procédé pour la préparation d'un peptide liant un émetteur selon l'une quelconque des revendications 1 à 8, comprenant l'immunisation d'un organisme approprié avec un émetteur, comprenant un colorant de type polyméthine qui est choisi dans le groupe formé par les colorants de type dicarbocyanine, tricarbocyanine, indotricarbocyanine, mérocyanine, styryle, squarilium et oxonol et les colorants de type rhodamine, phénoxazine ou phénothiazine.

14. Procédé pour la préparation d'un peptide liant un émetteur selon l'une quelconque des revendications 1 à 8, comprenant la préparation recombinante et/ou synthétique du peptide.

15. Utilisation d'un peptide liant un émetteur selon l'une quelconque des revendications 1 à 8, d'un acide nucléique selon la revendication 9 ou 10, d'une cellule hôte selon la revendication 11 ou d'un anticorps selon la revendication 12 comme agent diagnostique pour le diagnostic in vitro.

16. Kit diagnostique pour le diagnostic in vitro, comprenant au moins un agent choisi parmi un peptide liant un émetteur selon l'une quelconque des revendications 1 à 8, un acide nucléique selon la revendication 9 ou 10, une cellule hôte selon la revendication 11 ou un anticorps selon la revendication 12, le cas échéant ensemble avec d'autres adjuvants et/ou des indications, dans des récipients communs ou séparés.

17. Procédé pour la détermination quantitative in vitro d'une substance contenue dans un échantillon, comprenant l'étape
a) de mise en contact d'un peptide liant un émetteur selon l'une quelconque des revendications 1 à 8 avec un émetteur, l'interaction de l'émetteur du conjugué avec le peptide liant un émetteur provoquant une modification des propriétés spectrales d'émission de l'émetteur et
b) de mesure de la modification des propriétés spectrales d'émission de l'émetteur.

18. Procédé pour la détermination quantitative directe in vitro d'une substance contenue dans un échantillon ou d'un agent détectant un antigène présent dans l'échantillon selon la revendication 17, comprenant en outre l'étape
d) de quantification de la substance contenue dans l'échantillon au moyen de la modification mesurée des propriétés d'émission de l'émetteur.

19. Procédé selon la revendication 17 ou 18, où la modification des propriétés spectrales d'émission de la partie de l'émetteur est choisie parmi une modification du plan de polarisation, de la phosphorescence, en particulier de l'intensité de phosphorescence, de la durée de vie de la fluorescence et un déplacement bathochrome du maximum d'absorption et/ou de fluorescence.

20. Procédé selon l'une quelconque des revendications 17 à 19, où on met en contact avec l'échantillon comme peptide liant un émetteur des fragments d'anticorps, tels que par exemple des fragments Fab, des fragments scFv, des chaînes scTCR, des anticorps à chaîne simple et leurs mélanges.

21. Procédé selon l'une quelconque des revendications 17 à 20, où le peptide liant l'émetteur comprend une séquence selon la revendication 3.

22. Procédé selon l'une quelconque des revendications 17 à 21, où le peptide liant un émetteur présente une affinité de liaison avec l'émetteur de moins de 50 nM et de préférence de moins de 10 nM.
